(19) Europäisches Patentamt
European Patent Office
Office européen des brevets

(11) **EP 3 980 123 B1**

(12) **FASCICULE DE BREVET EUROPEEN**

(45) Date de publication et mention
de la délivrance du brevet:
**05.07.2023 Bulletin 2023/27**

(21) Numéro de dépôt: **21728208.6**

(22) Date de dépôt: **21.05.2021**

(51) Classification Internationale des Brevets (IPC):
**A61K 36/185** (2006.01)     **A61P 17/00** (2006.01)
**A61Q 19/00** (2006.01)     **A61K 8/49** (2006.01)
**A61K 8/9789** (2017.01)     **A61Q 1/02** (2006.01)
**A61Q 19/08** (2006.01)     **A61Q 19/10** (2006.01)

(52) Classification Coopérative des Brevets (CPC):
**A61P 17/00; A61K 8/4973; A61K 8/9789;
A61K 36/185;** A61K 2236/00; A61Q 1/02;
A61Q 19/08; A61Q 19/10

(86) Numéro de dépôt international:
**PCT/EP2021/063690**

(87) Numéro de publication internationale:
**WO 2021/234159 (25.11.2021 Gazette 2021/47)**

(54) **EXTRAIT DE GRAINES DE MORINGA PEREGRINA RICHE EN 2,5-DIFORMYLFURAN, SON PROCÉDÉ D'OBTENTION ET SON UTILISATION DANS DES COMPOSITIONS COSMÉTIQUES**

AN 2,5-DIFORMYLFURAN REICHES MORINGA-PEREGRINA-SAMENEXTRAKT, VERFAHREN ZU SEINER HERSTELLUNG UND SEINE VERWENDUNG BEI KOSMETISCHEN ZUSAMMENSETZUNGEN

MORINGA PEREGRINA SEED EXTRACT RICH IN 2,5-DIFORMYLFURAN, PROCESS FOR OBTAINING SAME AND USE THEREOF IN COSMETIC COMPOSITIONS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.05.2020 FR 2005429
17.03.2021 FR 2102686**

(43) Date de publication de la demande:
**13.04.2022 Bulletin 2022/15**

(73) Titulaire: **Agence Francaise pour le
Développement d'Alula
75008 Paris (FR)**

(72) Inventeurs:
• **DODINET, Elizabeth
12560 Saint-Laurent d Olt (FR)**
• **BOURGETEAU, Vincent
56130 Férel (FR)**

(74) Mandataire: **Loyer & Abello
9, rue Anatole de la Forge
75017 Paris (FR)**

(56) Documents cités:
**FR-A1- 2 776 519     FR-A1- 2 946 879**

• **MAHMOUD A. KOHEIL ET AL: "Anti-inflammatory
and antioxidant activities of Moringa peregrina
Seeds", FREE RADICALS AND ANTIOXIDANTS,
vol. 1, no. 2, 1 avril 2011 (2011-04-01), pages 49-61,
XP055753955, ISSN: 2231-2536, DOI:
10.5530/ax.2011.2.10**
• **SHAYMAA FADHEL ABBAS ALBA ET AL:
"Cytotoxic and Urease Inhibition Potential of
Moringa peregrina Seed Ethanolic Extract",
INTERNATIONAL JOURNAL OF
PHARMACOLOGY, vol. 15, no. 1, 15 décembre
2018 (2018-12-15), pages 151-155, XP055753970,
PK ISSN: 1811-7775, DOI:
10.3923/ijp.2019.151.155**

- MAGED MOHAMED MAHER ABOU-HASHEM ET AL: "Induction of sub-G0 arrest and apoptosis by seed extract of Moringa peregrina (Forssk.) Fiori in cervical and prostate cancer cell lines", JOURNAL OF INTEGRATIVE MEDICINE, vol. 17, no. 6, 1 novembre 2019 (2019-11-01), pages 410-422, XP055754018, ISSN: 2095-4964, DOI: 10.1016/j.joim.2019.09.004
- S MAJALI IBRAHIM ET AL: "Assessment of the antibacterial effects of Moringa peregrina extracts", AFRICAN JOURNAL OF MICROBIOLOGY RESEARCH, vol. 9, no. 51, 28 décembre 2015 (2015-12-28), pages 2410-2414, XP055754048, DOI: 10.5897/AJMR2015.7787

## Description

### Domaine technique

L'invention se rapporte au domaine de la cosmétique et nutricosmétique et plus particulièrement celui des ingrédients actifs entrant dans la formulation des compositions pour les soins de la peau. L'invention a trait à un extrait des graines de *Moringa peregrina* riche en composé 2,5-diformylfuran (DFF). L'invention concerne également le procédé d'obtention d'un extrait particulier des graines de *Moringa peregrina,* les compositions cosmétiques comprenant de tels extraits, et enfin l'utilisation cosmétique ou nutricosmétique de telles compositions pour le soin de la peau, du cuir chevelu et des phanères.

### Arrière-plan technologique

Les Moringaceae constituent une famille mono-générique (un seul genre, *Moringa* Adans), élément de la flore Saharo-sindienne, constituée d'entre douze et quatorze espèces selon les auteurs, réparties de l'Afrique orientale à l'Asie. Le genre est classiquement divisé en 3 sections qui ne sont, cependant, pas confirmées comme mono-phylétiques par les analyses phylogénétiques. Ces dernières ont plutôt mis en évidence des clades axés sur certains caractères morphologiques : pachycaules (« bottle trees ») ; tubéreux (« tuberous trees ») et ni pachycaules - ni tubéreux (en anglais « slender trees »). L'espèce *Moringa peregrina* (Forssk.) Fiori, appartient au troisième groupe. Les quelques études génétiques sur le genre ou la famille confirment la réalité de l'espèce par rapport aux autres espèces dans le genre, notamment vis-à-vis du Moringa indien, *Moringa oleifera* Lam. (voir notamment les articles: OLSON M. E. 2002, Combining Data from DNA Sequences and Morphology for a Phylogeny of Moringaceae (Brassicales), Systematic Botany 27(1): 55-73; HASSANEIN A. M. A. ET AL-SOQEE, A. A., 2018, Morphological and genetic diversity of Moringa oleifera and Moringa peregrina génotypes, Horticulture, Environment and Biotechnology 59(2): 251-261). Un article récent sur le *Moringa peregrina* échantillonné sur différentes localités en Arabie Saoudite a conclu, en utilisant des marqueurs ITS, à une stabilité génétique de l'espèce (ALAKLABI A., 2015, Genetic diversity of Moringa peregrina species in Saudi Arabia with ITS sequences, Saudi Journal of Biological Sciences 22: 186-190) avec cependant un haut niveau de variation génétique intra-populations.

L'espèce *Moringa peregrina* se retrouve dans les environnements rocheux du Yémen, d'Oman, et d'Arabie Saoudite, en Afrique orientale, au Soudan, en Éthiopie, Érythrée, Somalie et Djibouti. Sa présence en Iran semble limitée aux provinces sud-orientales mais demande à être confirmée (PROTA14 = MUNYANZIZA E. ET YONGAB1 K. A., Vegetable oils/Oléagineux, Moringa peregrina (Forssk.) Fiori, http://database.prota.org/protahtml/moringa peregrina _fr.htm, accès le 23/10/2019). Au Levant et en Égypte, l'espèce ne représente, aujourd'hui, plus que de rares stations dispersées, relictuelles (à l'exception de quelques peuplements altitudinaux), principalement dans les secteurs de l'aire soudanienne. *Moringa peregrina* est également considéré aujourd'hui comme rare et en danger au Soudan et au Yémen. *Moringa peregrina* occupe par rapport aux autres espèces de son clade les habitats les plus arides et les plus inhospitaliers. Il est apparemment plus résistant à la sécheresse que *Moringa oleifera* qui est planté commercialement à grande échelle dans les zones tropicales et subtropicales. Des travaux récents ont montré que la taille et la grosseur des graines impactait favorablement le temps de germination et le taux et la rapidité de la croissance des jeunes individus (GOMAA N. H. ET PICÓ F. X., 2011, Seed germination, seedling traits, and seed bank of the tree Moringa peregrina (Moringaceae) in a hyper-arid environment, American Journal of Botany 98(6): 1024-1030), indiquant un ajustement dans l'allocation des ressources sur la qualité de la graine plutôt que le nombre, ce qui permet à *Moringa peregrina* de se reproduire efficacement dans des environnements abiotiques extrêmes (hyperarides). Les graines de *Moringa peregrina* ont une mesotesta centrale plus épaisse, en termes de couche de cellules, que celles de *M oringaoleifera.*

Il existe quelques mentions historiques qui tendent à indiquer que l'huile de *Moringa peregrina* faisait l'objet d'un commerce actif dans les débuts de l'Islam dans la région d'AlUla (NASEEF, A.A.S., 1995, Al- 'Ula, A study of Cultural and Social Heritage*).* L'huile produite localement à partir de *Moringa peregrina* est aujourd'hui destinée principalement à l'autoconsommation ou aux marchés locaux. En Arabie Saoudite, les feuilles étaient utilisées traditionnellement en décoction en usage interne pour soigner le diabète, les maladies du colon, les maladies des yeux et les anémies (ABDEL-KADER M. S., HAZAZI A. M. A., ELMAKKI O. A. ET ALQASOUMI S. I., 2018, A survey on the traditional plants used in Al Kobah village, Saudi Pharmaceutical Journal 26(6): 817-821) et comme diurétique, rubéfiant et astringent (AQEEL A. A. M. , TARIQ M., M OSSA J. S., AL-YAHYA M. A. ET AL-SAIO M. S., 1984, « Plants used in Arabia Folk medicine », Report submitted to Saudi Arabian National Cenre for Science and Technology, Riyadh, Saudi Arabia). En Oman, l'huile, extraite par les femmes à la fin de l'été, est utilisée contre les migraines, la fièvre, les brûlures, les lacérations et les fractures, la constipation et les douleurs d'estomac, contre les douleurs musculaires et contre la sécheresse capillaire, contre les douleurs de l'enfantement (GHAZANFAR S. A., 1994, Handbook of Arabian Médicinal Plants, 1st éd., CRC Press, Boca Raton, Ann Harbor, s.u. ; GHAZANFAR S.A., 1998, Plants of Economic Importance, cap. 15, in G HAZANFAR, S.A. ET FISHER, M. (éd.) Végétation of the Arabian Peninsula. Geobotany 25, p. 241-264, Kluwer Academic Publishers, tableau 11.1, p. 247 et 11.7 p. 251). Elle était également utilisée dans des compositions parfumées (GHAZANFAR S.A., 1998, p. 259) et en Oman et au Yémen comme lotion faciale (GHAZANFAR S.A. ET RECHINGER B., 1996, Two multi-purpose seed oils form Oman. Plants for Food and Medicine. Paper presented at the joint meeting of the Society for Economic Botany and International Society for Ethnopharmacology, 1-7 July 1996, London).

On connaît des extraits provenant de la graine de *Moringa oleifera,* dans le domaine cosmétique. Par exemple on connaît du document FR296879 un extrait de graines entières (avec téguments) de *Moringa oleifera* contenant de l'huile (dont triglycérides, acides gras et lipides polaires) et des polyphénols et son utilisation dans des compositions cosmétiques pour lutter contre le vieillissement cutané. Dans ce document, c'est la partie apolaire de la graine de *Moringa oleifera* qui apparaît comme active, et plus particulièrement la partie huileuse. On connaît également du document FR2776519 que des extraits protéiques de graines de *Moringa oleifera,* connus pour leurs effets clarifiants sur les eaux turbides, présentent sur la peau et les muqueuses un effet adoucissant, conditionneur physiologique, hydratant, restructurant, réparateur et en tant qu'actif antipollution. Dans ce document les principes actifs sont des protéines de poids moléculaires compris entre 6 500 et 8 800 Da qui sont obtenues par extraction aqueuse sur le tourteau de *Moringa oleifera.* On connaît encore le document FR3076460 qui concerne l'utilisation d'un extrait protéique de graine non germée et déshuilée de *Moringa oleifera* pour le traitement des peaux et/ou muqueuses sensibles, sensibilisées, réactives, fragiles et/ou fragilisées et/ou dans le traitement et/ou la prévention de l'érythème, en particulier l'érythème fessier du nourrisson. Dans ce document, le procédé d'extraction permet d'obtenir une fraction majeure de protéines dont les poids moléculaires sont environ de 8800 Da. On connaît encore du document KR20130088224 l'utilisation d'un extrait de graine entière germée de *Moringa oleifera* en cosmétique, en particulier obtenu par une extraction à l'aide d'un fluide supercritique. Ce procédé permet d'isoler des caroténoïdes et acides aminés apolaires et qui sont décrits comme actifs pour une utilisation cosmétique blanchissante. Tous les documents précités concernent l'utilisation de l'espèce *Moringa oleifera,* aucun ne décrit l'utilisation dans le domaine cosmétique de l'espèce *Moringa peregrina.* Dans le document de Kolheil *et al.,* XP055753955, 2011, est divulgué sur les graines entières de *Moringa peregrina* l'extraction à l'éthanol de composés polyphénoliques bioactifs, de tanins, de flavonoides, de saponines, de stérols insaturés et/ou triterpènes. L'extrait obtenu est conservé à 4°C et présente un effet anti-oxydant. Dans le document d'Abbas Alba *et al.,* XP055753970, 2018, est divulgué sur les graines entières de *Moringa peregrina* une extraction éthanolique pendant trois jours et plus. La concentration des filtrats est obtenue sous pression réduite à une température comprise entre 45 et 50°C. Dans le document d'Abou-Hashem *et al.,* XP055754018, 2019, est divulgué sur les graines entières de *Moringa peregrina* une extraction éthanolique pendant trois fois 72 heures. L'extrait obtenu est ensuite filtré et concentré avec un évaporateur rotatif à une température d'environ 45°C. Dans le document de Majali Ibrahim *el al.,* XP055754048, 2015, est divulgué sur les graines entières de Moringa peregrina une extraction par l'éthanol sous agitation pendant une demi-heure puis la précipitation pendant 72 heures. Aucun des documents précités ne divulgue l'extraction éthanolique sur le tourteau de la graine non décortiquée de *Moringa peregrina.*

Plus spécifiquement pour l'espèce *Moringa peregrina,* on connaît que certains composés phénoliques et flavonoïdes obtenus de feuilles ou de la graine entière de *Moringa peregrina* présentent une activité anti-oxydante (AL-DABBAS M., 2017, Antioxidant activity of different extracts from the aerial part of Moringa peregrina (Forssk.) Fiori, from Jordan, Pakistan Journal of Pharmaceutical Sciences, 30(6): 2151-2157). Ces composés sont extraits avec des solvants tels que le méthanol, l'éthyl acétate ou l'hexane à partir des feuilles ou des graines entières. Il apparaît que ce sont les feuilles qui comprennent le plus de composés actifs.

Ainsi, selon l'espèce utilisée dans le genre *Moringa,* il est observé que selon la partie des plantes (feuille, graine), la partie des graines (entière ou non, décortiquée ou non) et le procédé d'extraction mis en oeuvre, notamment le choix du solvant, les molécules extraites s'avèrent être différentes. Or la composition de l'extrait conditionne l'activité biologique et en conséquence l'efficacité cosmétique.

Considérant ce qui précède, un problème que se propose de résoudre l'invention est de développer des produits nouveaux à base d'un extrait de l'espèce *M. peregrina* du genre Moringa qui soient utilisables en cosmétique et faciles à mettre en oeuvre.

De ce fait, la Demanderesse a mis en évidence un nouvel extrait obtenu à partir des graines et plus spécifiquement du tourteau des graines de l'espèce *Moringa peregrina,* qui présente notamment une activité relaxante et antistress de la peau, une activité anti-vieillissement ainsi qu'une activité préventive des tâches de vieillesse. L'extrait selon l'invention est riche en 2,5-diformylfuran (DFF), composé également nommé 2,5-furandicarboxaldéhyde. L'extrait est obtenu spécifiquement à partir des graines ou plus spécifiquement du tourteau des graines non décortiquées de *Moringa peregrina,* notamment par une extraction alcoolique. L'extrait selon l'invention est nouveau à double titre dans le domaine de la cosmétique par rapport aux extraits de l'art antérieur, d'une part par l'espèce spécifique d'origine utilisée et d'autre part par son contenu en composés particuliers.

Par accord intergouvernemental du 10 avril 2018 entre le gouvernement de la République française et le Royaume d'Arabie Saoudite, la demanderesse, Agence Française Pour Le Développement d'AlUla (AFALULA) ainsi que la Commission Royale pour AIUIA (RCU) ont pour projet commun notamment de développer une agriculture responsable et l'économie locale, notamment par la production locale de produits naturels dérivés des plantes indigènes et de protéger la biodiversité et les droits de la région d'AlUla du Royaume d'Arabie Saoudite. Le Royaume d'Arabie Saoudite est membre du Protocole de Nagoya depuis le 8 octobre 2020. Au moment de la rédaction du présent brevet, le règlement d'exécution en vertu duquel le Protocole de Nagoya sera intégré dans les aspects pertinents du droit local est en cours d'examen. Par conséquent, à ce stade, le Royaume d'Arabie Saoudite n'a pas d'exigences spécifiques en ce qui concerne cette demande de brevet et le Protocole de Nagoya. Ainsi, au jour du dépôt de la demande de brevet, il n'y a aucune exigence de certificat de conformité concernant l'accès aux ressources génétiques.

**Résumé**

L'invention a pour premier objet un extrait des graines de *Moringa peregrina* riche en composé 2,5-diformylfuran. Le composé 2,5-diformylfuran est un composé de nature osidique rare dans le règne végétal et synthétisé à partir du furfural via la synthèse intermédiaire de 5 hydroxyméthylfurfural.

Grâce à sa caractéristique, une concentration riche en 2,5-diformylfuran, l'extrait selon l'invention est unique dans le genre *Moringa.* Il sera démontré que l'espèce *Moringa peregrina* présente un profil moléculaire particulier et différent de ceux connus des autres espèces du genre, notamment de l'espèce *Moringa oleifera,* que la Demanderesse a su mettre en évidence.

L'invention a pour deuxième objet un procédé d'obtention d'un extrait des graines de *Moringa peregrina* selon l'invention, comprenant les étapes suivantes selon lesquelles :

a) on recueille les graines non décortiquées de *Moringa peregrina* que l'on sèche pour obtenir un taux d'humidité interne inférieur à 8%;
b) on presse les graines séchées de sorte à séparer l'huile du reste de la graine pour obtenir le tourteau,
c) on broie le tourteau obtenu à l'étape b),
d) on disperse le broyat obtenu à l'étape c) dans une proportion d'environ 25% en poids de matière solide par rapport au poids total mis en oeuvre dans un solvant majoritairement alcoolique, l'alcool étant choisi parmi l'éthanol ou le méthanol avec éventuellement un co-solvant de type polyol ou eau subcritique, dans une proportion de 80 à 100% en poids d'alcool par rapport au poids total du solvant ;
e) on réalise une extraction solide-liquide, sous agitation, à une température comprise entre 16 et 30°C pendant une durée d'environ 2heures,
f) on sépare les phases liquide et solide de manière à éliminer la phase solide et récupérer un extrait liquide de tourteau de *Moringa peregrina*, et
g) éventuellement, on sèche l'extrait de *Moringa peregrina* liquide obtenu de manière à obtenir un extrait de *Maringa peregrina* solide.

L'invention a pour troisième objet une composition cosmétique ou nutricosmétique comprenant à titre d'agent actif, une quantité efficace d'un extrait de graine de *Moringa peregrina* selon l'invention et un excipient physiologiquement acceptable.

L'invention a enfin pour quatrième objet l'utilisation cosmétique ou nutricosmétique d'une composition selon l'invention, pour améliorer l'aspect de la peau, des muqueuses ou des phanères, pour relaxer, apaiser et déstresser la peau et prévenir et/ou lutter contre les signes cutanés du vieillissement et/ou du photo-vieillissement, pour prévenir les tâches de vieillesse.

**Brève description des figures**

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif.

**Description des modes de réalisation**

Dans cette description, à moins qu'il n'en soit spécifié autrement, il est entendu que, lorsqu'un intervalle est donné, il inclut les bornes supérieures et inférieures dudit intervalle.

Dans la présente invention, les abréviations suivantes signifient :

- MTT : bromure de 3-(45-dimethylthiazol-2-yl)-2,5-diphenyl tétrazolium (test MTT est une méthode rapide pour la numération des cellules vivantes)
- SDS : Sodium Dodecyl Sulfate

- PBS : Phosphate Buffet- Saline
- ELISA : Enzyme-Linked Immunosorbent Assay
- PCR : Polymerase Chain Reaction
- ANOVA : Analysis Of Variance
- MSH : Mélanocyte Stimulating Hormone

Dans la présente invention, on entend par :

- « extrait riche en composé 2,5-diformylfuran » un extrait contenant une quantité de composé 2,5-diformylfuran supérieure aux autres ingrédients identifiés, soit une quantité supérieure à 50 % par rapport à la matière sèche de l'extrait total.
- « quantité efficace » la quantité nécessaire de molécules actives pour obtenir le résultat recherché, à savoir, permettre notamment d'obtenir une protection de la matrice extra-cellulaire de la peau.
- « application topique », le fait d'appliquer ou d'étaler le principe actif selon l'invention, ou une composition le contenant, à la surface de la peau, d'une muqueuse ou des phanères.
- « physiologiquement acceptable » qui convient à une utilisation topique, en contact avec la peau humaine, ou à une utilisation par d'autres voies d'administration, par exemple la voie orale ou l'injection dans la peau, sans risque de toxicité, d'incompatibilité, d'instabilité, de réponse allergique.
- « tourteau », la partie déshuilée de la graine après pressage. Il s'agit du résidu solide de l'extraction de l'huile des graines. C'est un coproduit de la trituration, procédé de fabrication de l'huile. Il représente généralement de 50 à 75% de la masse des graines.
- « graines non décortiquées », le fait que la coque, ou péricarpe, des graines récoltées soit maintenu autour des graines.
- « à maturité du fruit », le fait que le fruit soit mûr, préférentiellement lorsque la gousse est en début de déhiscence et qu'elle prend une coloration beige foncé à brune et lorsqu'une torsion exercée de 180° sur le quart inférieur de la gousse provoque l'amorce de l'ouverture des valves.
- « solvant majoritairement alcoolique », le fait que le solvant de type alcoolique puisse comprendre un co-solvant présentant les caractéristiques suffisantes pour extraire le principe actif, sachant que l'éthanol 96° pur apparaît être le solvant alcoolique le plus adapté.
- « environ » une marge plus ou moins de 10% à 20% par rapport à l'information donnée (durée, pourcentage..).
- « molécule active », également « principe actif », la molécule de 2,5-diformylfuran extraite selon le procédé de l'invention à partir des graines de *Moringa peregrina*. Cette molécule est responsable des activités biologiques décrites dans la présente invention.
- « agent actif » une quantité suffisante d'un extrait selon l'invention pour obtenir les activités biologiques décrites. Selon si l'extrait est liquide ou séché, concentré ou non, les quantités de l'agent actif peut varier dans les proportions de 0.002 à 20% en poids par rapport au poids total de la composition.
- « signes du vieillissement cutané », toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV).

L'invention concerne en premier objet un extrait de graines de *Moringa peregrina* riche en composé 2,5-diformylfuran. La molécule de 2,5-diformylfuran, également nommée 2,5-furandicarboxaldéhyde, n'a jamais été caractérisée dans un extrait des graines des espèces du genre *Moringa.* L'espèce *Moringa peregrina* pousse dans des climats très arides. Ainsi, sa faculté à résister à la sécheresse lui a permis d'acquérir des caractéristiques particulières uniques, que la Demanderesse a pu identifier par la mise en oeuvre d'un procédé d'extraction adapté sur les graines entières ou préférentiellement sur le tourteau des graines.

Dans le cadre de la présente invention, la partie de plante choisie est la graine de *Moringa peregrina.* Il est connu que les graines de *Moringa peregrina* sont utilisées pour l'extraction de leur huile appelée huile peregrina (nom INCI « Moringa peregrina seed oil »), utilisée régionalement pour l'autoconsommation ou dans des indications médicinales traditionnelles diverses. Le tourteau obtenu après que la graine ait été déshuilée, est un déchet actuellement utilisé pour la nourriture des animaux notamment.

Selon le premier objet de l'invention, l'extrait de *Moringa peregrina* est obtenu par extraction solide-liquide du tourteau des graines non décortiquées, sous agitation, dans une proportion d'environ 25% en poids de matière solide par rapport au poids total mis en oeuvre dans un solvant majoritairement alcoolique, l'alcool étant choisi parmi l'éthanol ou le méthanol avec éventuellement un co-solvant de type polyol ou eau subcritique, dans une proportion de 70 à 100% en poids d'alcool par rapport au poids total du solvant, à une température comprise entre 16 et 30°C pendant une durée d'environ 2 heures, et par séparation des phases liquide et solide de manière à éliminer la phase solide et récupérer un extrait liquide de graine de *Moringa peregrina,* ledit extrait étant riche en composé 2,5-diformylfuran. De préférence, l'extrait selon l'invention est obtenu du tourteau des graines récoltées à maturité du fruit de *Moringa peregrina* après extraction de l'huile *peregrina* (nom INCI « Moringa peregrina seed oil).

Il convient d'indiquer que le principe actif de l'extrait selon l'invention, à savoir le 2,5-diformylfuran, est une molécule de polarité mixte présentant une certaine fragilité. Ainsi, l'extrait obtenu à partir de la graine entière non décortiquée devra faire l'objet d'une extraction sélective pour obtenir une concentration élevée de principes actifs, en utilisant les solvants et co-solvants appropriés et une température ne dépassant pas 30°C.

Les co-solvants peuvent être par exemple les éthers de glycols (le mono- ou di-propylène glycol, le propanediol, et autres dérivés du propylène glycol, les dérivés d'éthylène ou de diéthylène glycol) le glycérol, le diméthyl éther isosorbide, les méthyl ou éthyl ou propyl esters d'acides gras ; le dicraprylyl carbonate, dicaprylyl éther, acétate ou proprionate d'alkyles, acétone, méthyl ou éthyl cétone, des monoterpènes tels que alpha-pinène ou limonène. Les proportions de ces co-solvants peuvent être mélangés au solvant primaire (ex. éthanol ou méthanol) de 0 à 30% (V/V).

Les conditions d'extractions peuvent être sous pression atmosphérique ou sous vide ou sous atmosphère inerte, mais préférentiellement à l'obscurité à une température comprise entre 16 et 30°C.

Dans un mode de réalisation préférentiel selon l'invention, l'extrait est obtenu du tourteau des graines non décortiquées par extraction solide-liquide, avec un solvant alcoolique éthanol 96°.

Dans encore un autre mode de réalisation, l'extrait liquide obtenu est séché de manière à obtenir un extrait sec du tourteau des graines de *Moringa peregrina* contenant plus de 50% en poids de 2,5-diformylfuran par rapport au poids total de la matière sèche.

L'extrait sec du tourteau des graines de *Moringa peregrina* contient plus précisément environ 55% en poids de 2,5-diformylfuran, 2.5% de furfural, 1.2% de myristate d'isopropyle, 4.7% d'acide palmitique, 11.1% d'acide oléique et 25.8% de triglycérides par rapport au poids total de la matière sèche.

L'invention a pour deuxième objet un procédé d'obtention d'un extrait de graines de *Moringa peregrina* selon l'invention comprenant les étapes suivantes selon lesquelles :

a) on recueille les graines non décortiquées de *Moringa peregrina* que l'on sèche pour obtenir un taux d'humidité interne inférieur à 8%;

b) on presse les graines séchées de sorte à séparer l'huile du reste de la graine pour obtenir le tourteau,

c) on broie le tourteau obtenu à l'étape b),

d) on disperse le broyat obtenu à l'étape c) dans une proportion d'environ 25% en poids de matière solide par rapport au poids total mis en oeuvre dans un solvant majoritairement alcoolique, l'alcool étant choisi parmi l'éthanol ou le méthanol avec éventuellement un co-solvant de type polyol ou eau subcritique, dans une proportion de 70 à 100% en poids d'alcool par rapport au poids total du solvant ;

e) on réalise une extraction solide-liquide, sous agitation, à une température comprise entre 16 et 30°C pendant une durée d'environ 2heures,

f) on sépare les phases liquide et solide de manière à éliminer la phase solide et récupérer un extrait liquide de tourteau de *Moringa peregrina,* et

g) éventuellement, lorsque l'alcool est l'éthanol, on sèche l'extrait de *Moringa peregrina* liquide obtenu de manière à obtenir un extrait de *Moringa peregrina* solide.

Dans un mode de réalisation préférentiel, on recueille les graines non décortiquées, c'est à dire dont on garde la coque, à maturité du fruit et préférentiellement lorsque la gousse est en début de déhiscence.

Dans un mode de réalisation préférentiel, on sèche les graines pour obtenir un taux d'humidité interne autour de 6%, le séchage se fera préférentiellement sur clayette ventilée à l'abri des rayons du soleil de préférence sous ombrière à l'air libre.

On broie alors les graines séchées extemporanément au pressage à froid qui permet de séparer mécaniquement l'huile peregrina (nom INCI « Moringa peregrina seed oil ») du reste de la graine compressée à savoir le tourteau.

Le tourteau est alors broyé mécaniquement avec tout type de broyeurs mécaniques tels que système à marteaux, à fléaux, à couteaux, à concassage/déchiquetage.

L'extraction s'effectue avantageusement toujours sous agitation permettant ainsi une dispersion et une homogénéisation du solide dans le liquide, améliorant la diffusion du soluté dans le solvant.

Pour extraire majoritairement le composé 2,5-diformylfuran d'intérêt, un solvant alcoolique de type éthanol à 96° sera préféré, mais le méthanol peut également être utilisé, avec un co-solvant de type polyol ou de l'eau subcritique. A l'issue de l'extraction, la matière végétale, résiduelle et épuisée en composé d'intérêt, est avantageusement séparée de la phase liquide par filtration clarifiante. De manière encore plus préférée le solvant est l'éthanol à 96°. On obtiendra avantageusement un extrait liquide à partir du tourteau des graines de *Moringa peregrina* comprenant entre 0.5% et 1.

6% de matière sèche, cette matière sèche étant composée d'au moins 50% de 2,5-diformylfuran ce qui correspond approximativement entre 0.25% et 0.8% en poids du poids total de l'extrait liquide.

Dans un mode de réalisation du procédé d'obtention selon l'invention, l'extrait de *Moringa peregrina* liquide obtenu est purifié par distillation, microfiltration, ultrafiltration et/ou nanofiltration pour concentrer l'extrait en composé 2,5-diformylfuran d'intérêt par rapport aux matières organiques également extraites, notamment au reste de la matière extraite comme les corps gras et dérivés également extraits. Ces étapes de purification permettent de concentrer le composé d'intérêt au dépend d'autres composés extraits tels que cités ainsi que du solvant.

Dans un autre mode de réalisation du procédé d'obtention selon l'invention, l'extrait liquide obtenu est séché de manière à obtenir un extrait sec du tourteau des graines de *Moringa peregrina* contenant plus de 50% en poids de composé 2,5-diformylfuran d'intérêt par rapport au poids total de la matière sèche extraite.

Selon un mode de réalisation avantageux de l'invention, lorsque le solvant est l'éthanol, l'extrait de graines de *Moringa peregrina* liquide obtenu est préférentiellement séché par exemple par atomisation, lyophilisation ou zéodratation de manière à obtenir un extrait de tourteau de graines de *Moringa peregrina* solide, l'éthanol étant évaporé. Le séchage peut être réalisé en présence d'un support organique tel que la maltodextrine, cyclodextrine ou inuline, ou en présence d'un support inorganique tel que phyllosilicate, magnésium silicate ou carbonate et ses sels.

L'invention concerne également l'extrait des graines de *Moringa peregrina* susceptible d'être obtenu par le procédé d'obtention selon l'invention.

L'invention a pour troisième objet une composition cosmétique ou nutracosmétique comprenant à titre d'agent actif, une quantité efficace d'un extrait de graines de *Moringa peregrina* selon l'invention, et un excipient physiologiquement acceptable.

La composition selon l'invention peut être formulée sous la forme de différentes préparations adaptées à une administration topique ou à une administration orale.

Selon une première variante, les différentes préparations sont adaptées à l'administration topique et incluent les crèmes, les émulsions huile dans eau et eau dans huile, les laits, les pommades, les lotions, les huiles, les baumes, les solutions aqueuses ou hydro-alcooliques ou glycoliques, les sérums, les poudres, les patchs, les sprays ou tout autre produit pour application externe tel que par, exemple, les dispositifs médicaux ou les produits de la cosméto-textile.

Selon une deuxième variante, les différentes préparations sont adaptées à une administration orale ; l'extrait végétal comprenant le composé actif 2,5-diformulfuran pouvant entrer soit dans une composition alimentaire soit dans un complément alimentaire. Le complément alimentaire peut se présenter sous forme de gélules ou de capsules molles de gélatine ou végétales dans le cadre de la présente invention. Ledit complément alimentaire peut alors contenir de 0.01 à 100% en poids de l'extrait végétal. Plus préférentiellement, la quantité en extrait végétal est de 0.02 à 40 % en poids, et en particulier de 0.2 à 20 % en poids par rapport au poids total de la composition.

Dans le cadre d'une utilisation alimentaire, à visée nutritive ou cosmétique (cosmeto-food ou nutricosmétique), la composition sera avantageusement formulée sous la forme d'une préparation adaptée à une administration orale. Elle pourra ne pas comprendre d'excipient et être constituée, en intégralité, de l'extrait végétal comprenant le composé actif 2,5-diformylfuran.

Selon un mode de réalisation préférentiel, les compositions selon l'invention sont destinées plus particulièrement à une administration par voie topique. Ces compositions doivent donc contenir un milieu cosmétiquement acceptable, c'est-à-dire compatible avec la peau et les phanères, et couvrent toutes les formes cosmétiques. Ces compositions pourront notamment être sous forme de crèmes, émulsions huile-dans-eau, ou eau-dans-huile ou émulsions multiples, sérums, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, émollients, épaississants, diluants, tensioactifs, anti-oxydants, agents bioactifs, colorants, conservateurs, parfums. Elles peuvent être utilisées comme produit de soin et/ou comme produit de maquillage de la peau.

La composition selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. La composition selon l'invention pourra avantageusement être utilisée dans les soins antipelliculaires. Elle peut également se présenter sous forme de teinture ou de mascara à appliquer au pinceau ou au peigne, en particulier sur les cils, les sourcils ou les cheveux.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook ("International Nomenclature of Cosmetic Ingrédients (13ème Ed. 2010) publié par "the Personal Care Products Council, Inc.", Washington, D.C.) décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

Dans tous les cas, l'homme de métier veillera à ce que ces adjuvants ainsi que leurs proportions soient choisis de telle manière à ne pas nuire aux propriétés avantageuses recherchées de la composition selon l'invention.

Selon un mode de réalisation avantageux de l'invention, la quantité ou teneur en extrait végétal dans la composition selon l'invention est de 0.002 à 20 % en poids, et en particulier de 0.001 à 10 % en poids par rapport au poids total de la composition.

Enfin, l'invention a pour quatrième objet l'utilisation cosmétique ou nutricosmétique d'une composition selon l'invention pour améliorer l'aspect de la peau, des muqueuses et des phanères, pour relaxer, apaiser et déstresser la peau et prévenir et/ou à lutter contre les signes cutanés du vieillissement et/ou du photo-vieillissement et prévenir les tâches de vieillesse.

Selon un mode de réalisation, l'utilisation selon l'invention a plus particulièrement pour but de relaxer, apaiser et déstresser la peau et lutter contre les signes du vieillissement cutané.

Selon un autre mode de réalisation, l'utilisation selon l'invention a pour but d'éviter l'apparition des tâches de vieillesse.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Exemples

Exemple 1 : Préparation d'un extrait végétal à partir du tourteau de *Moringa peregrina*

Des graines non décortiquées et récoltées à maturité du fruit de *Moringa peregrina* (Forssk.) Fiori ont été séchées pour obtenir un taux d'humidité interne inférieur à 8% et préférentiellement autour de 6%, puis pressées avec une presse mécanique à vis sans fin, de sorte à séparer l'huile du reste de la graine pour obtenir d'une part l'huile vierge et d'autre part un tourteau. Le tourteau est alors isolé sous forme de boudins prédécoupés en morceau de 1 à 2 cm. A partir du tourteau préchauffé 10 mn à 55°C, on effectue une macération et extraction avec de l'éthanol à 96° préchauffé 10 mn à 55°C dans le ratio 25/75% (m/m), on cisaille au mixer pendant 15 mn puis on laisse agiter à l'hélice pendant 2 h à température comprise entre 16°C et 30°C. On filtre alors au Büchner sous vide et on obtient un filtrat jaune pâle contenant 1,15% de matière sèche. L'extrait liquide obtenu est nommé par la suite « extrait *peregrina* selon l'invention » ou « extrait *peregrina* » ou « extrait de tourteau de *peregrina* ». Cet extrait liquide est utilisé par la suite dans les différents tests d'efficacité.

Cet extrait *peregrina* selon l'invention contient 1,15% de matière sèche comportant lui-même (résultats exprimés par rapport à la matière Sèche (MS) :

[Tableaux1]

| Composés actifs | Composés totaux MS | | % |
|---|---|---|---|
| | Furfural | 2.528 | 57.18 |
| | 2,5-Furandicarboxaldéhyde | 54.66 | |
| Corps gras d'origine végétale | Myristate d'isopropyle | 1.175 | 42.812 |
| | Acide palmitique | 4.713 | |
| | Acide oléique | 11.093 | |
| | Trigycérides | 25.831 | |

L'extrait sec décrit ci-dessus est obtenu par une méthode gravimétrique basée sur la masse avant et après évaporation présent dans l'extrait liquide.

Le 2,5-Furandicarboxaldehyde ou 2,5-diformylfuran est le principe actif de cet extrait.

Il a été dosé par méthode chromatographique, et plus précisément par chromatographie gazeuse couplée à un détecteur à ionisation de flamme.

Le Furfural a été dosé selon une méthode identique

Le Myristate d'isopropyle a été dosé selon une méthode identique

L'acide palmitique et oléique ont été dosés selon une méthode identique

Les triglycérides ont été isolés par ultra-centrifugation.

Exemple 2 : Effet de l'extrait *peregrina* selon l'invention comme anti-oxydant

L'objectif de cette étude est d'évaluer la modulation de l'activité anti-oxydante par l'extrait *peregrina* dans un modèle colorimétrique *in vitro* acellulaire faisant appel au radical DPPH (2,2-diphenyl-1-picrylhydrazyl) ainsi qu'à l'antioxydant de référence, l'acide ascorbique. La méthode utilisée est dite d'inhibition. Elle est basée sur la dégradation du radical oxydant DPPH, de coloration violette absorbant à 540 nm, par un antioxydant de référence, l'acide ascorbique. Cette réaction sert de témoin positif et conduit à la formation du composé DPPH qui est incolore voire jaune clair. L'extrait *peregrina* selon l'invention et le produit de référence « Acide Ascorbique » sont mis en contact avec la solution de DPPH pendant 30 mn à 40°C. L'activité antioxydante est alors évaluée par mesure d'absorbance à 540 nm. La modulation de cette activité est exprimée en pourcentage de stimulation de l'activité antioxydante par l'actif testé, avec pour référence l'activité antioxydante maximale obtenue en présence d'acide ascorbique (T+).

Protocole : Une solution de DPPH est incubée, pendant 30 minutes à 40°C, en absence (contrôle), en présence de l'extrait de *peregrina* selon l'invention (T+) et à des concentrations décroissantes de l'échantillon à tester. A la fin de la période d'incubation, l'activité antioxydante en présence du produit de référence et en présence ou en absence de l'extrait *peregrina* a été révélée par coloration après 30 minutes à 40°C. Elle a ainsi été évaluée par mesure d'absorbance du milieu réactionnel à 540 nm. Pour chaque concentration testée, la modulation de l'activité antioxydante par le produit testé est calculée selon la formule suivante.

[Math. 1]

$$\text{Pourcentage de modulation de l'activité antioxydante} = 100 \times [(\text{DO540 Contrôle} - \text{DO540 Produit à tester}) / \text{DO540 Produit de référence}].$$

Si le résultat est négatif, le produit à tester sera considéré comme oxydant ; si le résultat est positif, le pourcentage sera exprimé en stimulation de l'activité anti-radicalaire. Les résultats obtenus sont donnés ci-après.

[Tableaux2]

|  |  | Inhibition DPPH |
|---|---|---|
| Extrait *peregrina* selon l'invention | 2% | 36 |
|  | 1.0% | 23 |
|  | 0.1% | 5 |

Conclusion : l'extrait *peregrina* selon l'invention est capable de protéger des radicaux libres, il présente des propriétés anti-oxydantes significatives dès la concentration de 1%.

Exemple 3 : Effet de l'extrait *peregrina* selon l'invention comme inhibiteur des métal loprotéases

L'objectif de cette étude est d'évaluer la modulation de l'activité inhibitrice des métalloprotéases par l'extrait *peregrina* selon l'invention dans un modèle *in vitro* acellulaire faisant appel à une collagénase de type I et une hyaluronidase, un complexe de substrat et un chromophore, Ninhydrine. Une solution tamponnée de collagénase de type I et hyaluronidase réagit avec un complexe de substrat spécifique et le transforme pour former un composé capable d'activer un chromophore par incubation à 80°C pendant 15 mn. L'activité de la collagénase et de la hyaluronidase peuvent ainsi être évaluées par mesure de l'absorbance à 565 nm. L'échantillon est mis en contact avec la solution de collagénase et hyaluronidase en même temps que le complexe de substrat des enzymes à 37°C pendant 5 minutes. Le substrat transformé par les enzymes est capable d'activer le chromophore par incubation à 80°C pendant 15 minutes. L'activité de la collagénase et hyaluronidase en présence/absence de l'échantillon est alors évaluée par mesure de l'absorbance à 565 nm. La modulation de cette activité est exprimée en pourcentage d'inhibition ou d'activation de l'activité de la collagénase et hyaluronidase en absence d'actif, c'est-à-dire uniquement en présence du substrat de l'enzyme.

Protocole : Une solution d'enzymes collagénase de type I et hyaluronidase est incubée dans son substrat, pendant 5 minutes, en absence ou présence de l'extrait *peregrina* selon l'invention testé. Les solutions sont ensuite mises en contact avec le chromogène, Ninhydrin, avant une incubation de 15 minutes à 80°C. A la fin de la période d'incubation, l'activité des enzymes collagénase et hyaluronidase avec et sans produit à l'essai ou de référence a été évaluée par mesure de l'absorbance des milieux réactionnels à 565 nm. Pour chaque concentration testée, la modulation de l'activité des enzymes collagénase et hyaluronidase par le produit à l'essai est calculée selon la formule suivante.

[Math. 2]

Pourcentage de modulation de l'activité des enzyme collagénase/ hyaluronidase =

100 x [(DO produit à l'essai ou de référence – DO collagénase/hyaluronidase

seule)/DO collagénase/hyaluronidase seule].

Si le résultat est négatif, le pourcentage est exprimé en inhibition des enzymes ; si le résultat est positif, le pourcentage est exprimé en activation des enzymes. Les résultats de l'inhibition des métalloprotéases par l'extrait *peregrina* selon l'invention sont donnés ci-dessous.

[Tableaux3]

|  |  | Inhibition versus Contrôle (%) |
|---|---|---|
| Extrait *peregrina* selon l'invention | 1% | 87 |
|  | 0.5% | 87 |
|  | 0.1% | 88 |
|  | 0.01% | 62 |

Conclusion : L'extrait *peregrina* selon l'invention engendre une forte inhibition des métalloprotéases (collagénase / hyaluronidase) de 62% aux taux très bas de 0.01%. L'extrait *peregrina* selon l'invention est capable d'inhiber fortement ces métalloprotéases et a un bon potentiel pour protéger avec une grande efficacité la matrice extra-cellulaire de la peau et, par cette inhibition, il montre un effet anti-vieillissement.

Exemple 4 : Effet de l'extrait *peregrina* selon l'invention pour inhiber les enzymes Histones Desacétylases (HDACs) et Sirtuine I

L'objectif de cette étude est de démontrer l'activité inhibitrice de l'extrait *peregrina* selon l'invention sur les enzymes HDACs et Sirtuines I. Une solution tamponnée de HDACs & Sirtuine I réagit avec un substrat pendant 20 minutes à 37°C et le transforme pour former un composé qui se colore en présence d'un développeur après incubation à 37°C pendant 10 minutes. L'activité maximum de désacétylation des Sirtuines peut ainsi être évaluée par mesure de l'absorbance à 405 nm. L'extrait *peregrina* selon l'invention ou le produit de référence « Trichostatin A (STA) inhibiteur 1 $\mu$M » sont mis en contact avec la solution de Sirtuines en même temps que le substrat de l'enzyme pendant 20 minutes à 37°C, le substrat transformé par l'enzyme est coloré par l'ajout d'un développeur. L'activité désacétylante des HDACs et Sirtuine I en présence de l'actif est alors évaluée par mesure de l'absorbance à 405 nm. La modulation de cette activité est exprimée en pourcentage d'inhibition ou d'activation de l'activité maximum des HDACs & Sirtuine I en absence d'actif, c'est-à-dire uniquement en présence du substrat des enzymes HDACs & Sirtuine I.

Protocole : Une solution d'enzymes Sirtuines est incubée dans son substrat pendant 20 mn en absence (contrôle) ou présence du produit de référence, ou de concentrations croissantes des produits à l'essai. L'extrait *peregrina* selon l'invention est testé aux concentrations suivantes : 2% ; 1% ; 0.1% (V/V). A la fin de la période d'incubation, l'activité des enzymes Sirtuines avec et sans produit à l'essai ou de référence a été révélée par coloration à l'aide d'une solution de développeur (10 mn à 37°C) et évaluée par mesure de l'absorbance des milieux réactionnels à 405 nm. Pour chaque concentration testée, la modulation de l'activité désacétylante des enzymes Histones Désacétysases et Sirtuines I par le produit à l'essai est calculée selon la formule suivante.

[Math. 3]

Pourcentage de modulation de l'activité des enzymes Sirtuines = 100 x [(DO$_{405}$ produit à l'essai ou de référence) – (DO$_{405}$ HDACs & Sirtuine I seules)]/DO$_{405}$ Sirtuines seules.

Si le résultat est négatif, le pourcentage est exprimé en inhibition de la réaction enzymatique ; si le résultat est positif, le pourcentage est exprimé en activation de la réaction enzymatique. Les résultats de l'inhibition des enzymes Histones Desacétylases (HDACs) sont donnés ci-dessous.

[Tableaux4]

| Extrait *peregrina* selon l'invention | pourcentage | Inhibition versus Contrôle (%) |
|---|---|---|
| | 2% | 20 |
| | 1% | ns |
| | 0.10% | -18 |

Conclusion : A 2% l'extrait *peregrina* selon l'invention montre une inhibition significative des HDACs ; cette inhibition signifie la capacité de promouvoir l'autoprotection des cellules de la peau contre la dérive génétique, notamment liée au processus de vieillissement. Ainsi l'extrait apparaît être utile contre l'une des dérives génétiques les plus courantes à la surface de la peau à savoir la fibrose qui se manifeste par l'apparition de « bouton » de chair (protubérance fibrotique). L'extrait pourra avantageusement interférer contre les phénomènes de fibrose à la surface de la peau et prévenir ainsi le vieillissement de la peau.

Exemple 5 : Effet de l'extrait *peregrina* selon l'invention pour moduler l'activité anti-inflammatoire de l'enzyme phospholypase-A2.

L'objectif de cette étude est d'évaluer la modulation de l'activité anti-inflammatoire de l'enzyme phospholipase A2 par un ou plusieurs échantillons dans un modèle In Vitro acellulaire faisant appel au kit d'analyse « SPLA2 (type V) Inhibitor Screening Assay Kit ». La Phospholipase A2 est une enzyme clé en amont du processus inflammatoire qu'enclenche la cascade arachidonique. Une solution tamponnée de Phospholipase A2 réagit avec un substrat spécifique, le diheptanoyl thio- PC, et le transforme pour former un composé qui se lie à un chromogène, le DTNB sous agitation à température ambiante. L'activité de la phospholipase A2 peut ainsi être évaluée par mesure de l'absorbance à 413 nm. L'extrait *peregrina* selon l'invention ou le produit de référence inhibiteur « Thioetheramide-PC » sont mis en contact avec la solution de Phospholipase A2 en même temps que le substrat de l'enzyme. Le substrat transformé par l'enzyme est coloré à l'aide du chromogène DTNB par agitation à température ambiante. L'activité de l'extrait *peregrina* selon l'invention ou du produit de référence est alors évaluée par mesure de l'absorbance à 413 nm. La modulation de cette activité est exprimée en pourcentage d'inhibition ou d'activation de l'activité de la Phospholipase A2 en absence d'actif, c'est-à-dire uniquement en présence du substrat de l'enzyme (diheptanoyl thio-PC).

Protocole : Une solution d'enzyme Phospholipase A2 est incubée dans son substrat, diheptanoyl thio-PC, en absence ou présence de l'inhibiteur de référence et de l'extrait *peregrina* selon l'invention, testé aux concentrations suivantes : 2% ; 1% ; 0.1% (V/V), puis le chromogène DTNB est incorporé avant une incubation de 15 minutes à 25°C. A la fin de la période d'incubation, l'activité de l'enzyme Phospholipase A2 avec et sans produit à l'essai ou de référence est évaluée par mesure de l'absorbance des milieux réactionnels à 413 nm. Pour chaque concentration testée, la modulation de l'activité de l'enzyme Phospholipase A2 par le produit à l'essai est calculée selon la formule suivante.

[Math. 4]

Pourcentage de modulation de l'activité de l'enzyme Phospholipase A2 = 100 x [(DO$_{405}$ produit à l'essai ou de référence – DO$_{405}$ sPLA2 seule) /DO$_{405}$ sPLA2 seule].

Si le résultat est négatif, le pourcentage est exprimé en inhibition de l'enzyme ; si le résultat est positif, le pourcentage est exprimé en activation de l'enzyme. Les résultats de la modulation de l'activité anti-inflammatoire de l'enzyme phospholypase-A2 sont donnés ci-dessous.

[Tableaux5]

|  |  | Inhibition Versus Contrôle (%) |
|---|---|---|
| Extrait *peregrina* selon l'invention | 2% | 19 |
|  | 1% | 16 |
|  | 0.10% | 11 |

Conclusion : L'extrait *peregrina* selon l'invention engendre une légère inhibition stable de la PLA2 dès la dose de 0.1% mais plus préférentiellement à 1 ou 2%. Cela signifie que l'extrait *peregrina* selon l'invention a la capacité de réduire très en amont la cascade arachidonique / cascade de l'inflammation; cet extrait présente ainsi un bon potentiel apaisant ou relaxant sur la peau.

Exemple 6 : Effet de l'extrait *peregrina* selon l'invention pour inhiber l'action de l'endothéline 1

L'endothéline est le vaso-constricteur le plus puissant connu dans le corps humain. D'un autre côté, la diminution de l'endothéline est également connue pour créer un effet vasodilatateur [HIRATA Y. et al., 1988, Cellular mechanism of action by a novel vasoconstrictor endothelin in cultured rat vascular smooth muscle cells, Biochemical and Biophysical Research Commununication 154: 3, p. 868-875,] [SHALINKUMAR P. et al., 2018, H2S Médiates the Vasodilator Effect of Endothelin-1 in the Cérébral Circulation. American Journal of Physiology. Heart and Circulatory Physioliogy 315, p. 1759-1764].

L'objectif est de doser l'endothéline de type 1 dans les cellules endothéliales micro-vasculaires humaines après exposition pendant 24h à l'extrait *peregrina* selon l'invention.

Protocole : Les cellules endothéliales micro-vasculaires humaines ont été fournies par la société PELOBiotech et mise en culture dans les plaques 96 puits selon les procédures de production du fournisseur. Il s'agit de laisser agir les extraits à différentes concentrations sur les cellules endothéliales à 80% de confluence pendant 24 heures, puis de quantifier l'endothéline 1 à l'aide du kit ELISA PicoKine (EDN1) dans les surnageants cellulaires. Un test de viabilité au préalable est réalisé pour définir les doses non-toxiques à utiliser lors de dosage de l'endothéline 1. Le contrôle négatif est réalisé à l'aide des cellules en milieu de culture sans traitement. Le contrôle positif dans le test de viabilité est le SDS à 0,5%. Toutes les conditions sont préparées dans des milieux de culture, et les cellules sont ensuite incubées à 36.5°C / 5% $CO_2$ pendant 24 heures.

a) Application des solutions à tester sur les cellules endothéliales :
Les produits à tester sont mis en contact avec les cellules endothéliales en sub-confluence dans les plaques 96 puits. Pour chaque concentration le test est réalisé sur 3 puits. Les plaques sont incubées pendant 24 heures ± 1 heure à 36,5°C / 5% $CO_2$.
b) Test de viabilité :
La viabilité cellulaire est évaluée par la méthode MTT sur les cellules après incubation avec les produits. Après 24 heures d'incubation, les surnageants sont récupérés et conservés à -20°C pour les dosages. Les puits sont ensuite rincés 1 fois avec 200 µL de PBS. Dans chaque puits 50 µL de solution de MTT à 0,5 mg/ml sont ajoutés : incubation pendant 3 heures à 36,5°C / 5% $CO_2$. Dans chaque puits 100 µL d'isopropanol sont ajoutés. Après homogénéisation, une lecture de l'absorbance est réalisée à 550 nm. Pour chaque condition, le rapport de la moyenne des densités optiques des cellules sur la moyenne des densités optiques des contrôles négatifs déterminera le taux de viabilité.
c) Dosage de l'endothéline 1 :
Le dosage est réalisé à l'aide du kit ELISA.

[Tableaux6]

| | Extrait concentration | Croissance cellulaire versus contrôle (%) | Endothelin 1 versus contrôle (%) | Endothelin 1 versus contrôle ( *pg/ml*) |
|---|---|---|---|---|
| Extrait *peregrina* selon l'invention | 5% | -22.31 | -53.21 | -71.8 |
| | 1% | 0 | -11.61 | -15.66 |
| | 0.10% | -5.77 | -25.11 | -33.88 |

Conclusion : Le test de viabilité réalisé à la fin de traitement n'a pas montré d'effet toxique pour les concentrations testées. Le dosage de l'endothéline 1 est réalisé dans les surnageants cellulaires à des concentrations non toxiques. La quantité de l'endothéline 1 pour chaque condition est dosée à l'aide du kit ELISA. Pour les cellules du contrôle négatif, les valeurs sont de l'ordre de 134.94 pg/ml. Pour les cellules traitées avec différentes concentrations d'extraits, les valeurs sont de 63.14 pg/ml (avec 5% de l'extrait selon l'invention) à 101.06 pg/ml (avec 0.1% de l'extrait selon l'invention), ce qui montre des inhibitions très significatives dès 0.1% de l'extrait selon l'invention avec environ 25% d'inhibition de production d'endothéline de type 1 et jusque 53% d'inhibition avec 5% de l'extrait selon l'invention.

Exemple 7 : Effet de l'extrait *peregrina* selon l'invention pour stimuler l'activité de la télomérase

Les télomères sont des complexes protecteurs de l'ADN situés à l'extrémité des chromosomes linéaires qui favorisent la stabilité chromosomique. La brièveté des télomères chez les êtres humains est en train de devenir un marqueur pronostique du risque et de la progression de la maladie, et de la mortalité prématurée dans de nombreux types de cancers, notamment le sein, la prostate, le côlon, la vessie, la tête et le cou, le poumon et les cellules rénales [ORNISH D., 2008, Increased Telomerase Activity and Comprehensive Lifestyle Changes: a Pilot Study, Lancet Oncology 9, p. 1048-1057]. Le raccourcissement des télomères est contrecarré par l'enzyme cellulaire télomérase.

L'objectif de l'étude est d'évaluer l'effet du composé nommé « Extrait *peregrina* selon l'invention » sur l'activité télomérase dans un modèle composé de kératinocytes humains adultes à faible niveau de passage en culture monocouche.

Protocole : Les kératinocytes humains ont été obtenus d'un donneur de 49 ans. Pour effectuer les expériences, les kératinocytes ont été utilisés à un faible niveau de passage (c.-à-d. passage n° 2 de l'isolement des cellules). Les cellules ont été cultivées en monocouche jusqu'à atteindre environ 75% de confluence avant d'être utilisées dans l'expérience.

Produit de référence : Le FK228 à 100 ng / ml a été utilisé comme inducteur de référence de l'activité télomérase1.

Protocole d'incubation : Les cellules ont été incubées pendant 24 heures en absence (contrôle) ou en présence de référence produit ou de concentrations croissantes de composés d'essai tel que l'extrait *peregrina* selon l'invention à : 0.5; 1 et 5% (v / v).

L'extrait *peregrina* selon l'invention est dilué directement dans le milieu d'incubation pour atteindre les différentes concentrations décrites ci-dessus.

Évaluation des effets :

- Mesure des protéines
  À la fin de la période d'incubation, les protéines totales des cellules ont été extraites des cellules et mesurées à l'aide d'une méthode spectro-colorimétrique (méthode de Bradford). Cette mesure permet de déterminer le volume exact d'extrait à utiliser pendant la mesure d'activité télomérase, afin de maintenir la même quantité de protéines (contenant télomérase) pour toutes les conditions testées au niveau de l'étape de PCR.
- Mesure de l'activité télomérase
  À la fin de la période d'incubation, la télomérase a été extraite des cellules et son activité a été déterminée à l'aide d'un kit spécifique et sensible. Le principe du kit de telomerase est de mesurer l'activité télomérase couplant une étape de PCR (dans laquelle la télomérase fonctionne sur son activité d'allongement) avec une étape ELISA permettant la détermination semi-quantitative de quantités de produit d'allongement de télomérase.
- Statistiques
  Les résultats sont exprimés en unités arbitraires pour le niveau d'activité de la télomérase (moyenne $\pm$ S.D). Le niveau de signification entre « véhicule » et « produit de référence » a été évalué en utilisant un test t de Student (*: $p < 0.05$). Le niveau de signification entre « contrôle » et « composé d'essai » a été évalué indépendamment pour chaque produit par une analyse de variance à un facteur (ANOVA unidirectionnelle) suivi d'un test Holm-Sidak (*:

p <0.05).

L'extrait *peregrina* selon l'invention, testé à 0.5% et 1 % (v /v), n'a pas modulé de manière significative l'activité de la télomérase par rapport à « Contrôle ». Testé à 5% (v / v), l'extrait *peregrina* selon l'invention a augmenté de manière significative l'activité de la télomérase de 18.9% (p<0.001), en comparaison avec « Contrôle ». Le produit de référence, nommé « FK228 », testé à 100 ng/ml, a significativement augmenté l'activité télomérase de 28.0% (p <0.01). Ce résultat était attendu et valide l'expérience. Les résultats de la stimulation de l'activité de la télomérase sont donnés ci-dessous.

[Tableaux7]

|  | Concentration de l'extrait | Croissance cellulaire versus contrôle (%) | Activité de la Telomerase versus contrôle (%) |
|---|---|---|---|
| Extrait *peregrina* selon l'invention | 5.00% | +5.8 | +18.90 |
|  | 1.00% | ca. -2.2 | +4.00 |
|  | 0.50% | -2.6 | +2.30 |

Conclusion l'extrait *peregrina* selon l'invention, testé à 0.5% et 1 % (v /v), n'a pas modulé de manière significative l'activité de la télomérase par rapport à « Contrôle ». Testé à 5% (v/v), l'extrait *peregrina* selon l'invention a augmenté de manière significative l'activité de la télomérase de 18.9% (p<0.001) en comparaison avec « Contrôle ». L'extrait selon l'invention agit directement sur cette voie enzymatique qui construit du télomère protecteur aux extrémités des chromosomes et ralentit le vieillissement naturel du matériel génétique. L'extrait selon l'invention permet donc de produire un effet anti-vieillissement sur les chromosomes.

Les exemple 2 à 7 permettent de démontrer que l'extrait *peregrina* selon l'invention présente des propriétés antivieillissement, anti-stress et relaxante qui démontre un profil d'un bon protecteur de la peau.

Exemple 8 : Effet de l'extrait *peregrina* selon l'invention pour stimuler l'activité de la tyrosinase

L'objectif de cette étude est d'évaluer l'activité sur l'enzyme Tyrosinase de l'extrait *peregrina* selon l'invention dans un modèle *in vitro* acellulaire faisant appel à une enzyme tyrosinase d'origine fongique (Sigma Aldrich réf. T3824), à son substrat L-Tyrosine (Sigma Aldrich réf. T3754) et un inhibiteur de référence l'hydroquinone (Sigma Aldrich réf.H17902, Inhibiteur = Hydroquinone 2.5 mM). Une solution tamponnée de Tyrosinase réagit avec un substrat L Tyrosine 2.5 mM pendant 60 minutes à 23°C et le transforme pour former un composé coloré. L'activité maximum de la Tyrosinase peut ainsi être évaluée par mesure de l'absorbance à 475 nm. L'extrait *peregrina* selon l'invention ou le produit de référence « Hydroquinone » sont mis en contact avec la solution de Tyrosinase en même temps que le substrat de l'enzyme pendant 60 minutes à 23°C, le substrat transformé par l'enzyme est naturellement coloré. L'activité de la Tyrosinase en présence de l'actif est alors évaluée par mesure de l'absorbance à 475 nm. La modulation de cette activité est exprimée en pourcentage d'inhibition ou d'activation de l'activité maximum de la Tyrosinase en absence d'actif, c'est-à-dire uniquement en présence du substrat de l'enzyme (L Tyrosine).

Protocole : Une solution d'enzyme Tyrosinase est incubée dans son substrat L Tyrosine pendant 60 minutes en absence (contrôle) ou présence du produit de référence, ou de concentrations croissantes de l'extrait *peregrina* selon l'invention / concentrations ; 2% ; 1% ; 0.1% (V/V). A la fin de la période d'incubation, l'activité de l'enzyme Tyrosinase avec et sans produit à l'essai ou de référence a été évaluée par mesure de l'absorbance des milieux réactionnels à 475 nm. Pour chaque concentration testée, la modulation de l'activité de l'enzyme Tyrosinase par le produit à l'essai est calculée selon la formule suivante :

[Math. 5]

$$\text{Pourcentage de modulation de l'activité de l'enzyme Tyrosinase} = 100 \times [(\text{DO475 produit à l'essai ou de référence}) - (\text{DO475 Tyrosinase seule})] / \text{DO475 Tyrosinase seule.}$$

Si le résultat est négatif, le pourcentage est exprimé en inhibition de l'enzyme ; si le résultat est positif, le pourcentage est exprimé en activation de l'enzyme. Les résultats de stimulation sur l'activité de la tyrosinase sont donnés ci-dessous.

[Tableaux8]

|  |  | Activation Versus Contrôle (%) |
|---|---|---|
| Extrait *peregrina* selon l'invention | 2% | 65 |
|  | 1% | 37 |
|  | 0.10% | 8 |

Conclusion : L'extrait *peregrina* selon l'invention est capable d'abaisser le taux d'activité basale de la tyrosinase, ce qui permet d'indiquer que cet extrait a la capacité d'augmenter une des protections naturelles de la peau, la protection contre les rayons ultraviolets.

Exemple 9 : Effet de l'extrait *peregrina* selon l'invention pour inhiber la production de mélanine

L'objectif de cette étude sur cultures cellulaires humaines est de regrouper l'ensemble des données utilisées, ainsi que les résultats obtenus, pour la réalisation du test de modulation de la mélanine sur les mélanocytes humains après exposition pendant 5 jours à l'extrait *peregrina* selon l'invention.

Protocole : Les mélanocytes humains sont mis en culture dans les plaques 96 et 24 puits.

Il s'agit de laisser agir l'extrait *peregrina* selon l'invention à des concentrations de 5% ; 2% ; 1% et 0.1% sur les mélanocytes à confluence pendant 5 jours. Un pré-test de viabilité au MTT après 24 heures permet d'évaluer la cytotoxicité et de choisir les concentrations pour le test de modulation de la mélanine. Cette modulation est évaluée par le dosage de la mélanine dans les lysats cellulaires après 5 jours d'exposition aux extraits. Le contrôle négatif est réalisé à l'aide des cellules en milieu de culture sans traitement. Le contrôle positif pour le test de viabilité est le SDS à 0.5%. Pour le test de la modulation de la mélanine des milieux avec et sans $\alpha$-MSH sont utilisés comme contrôles négatifs.

Toutes les conditions sont préparées dans des milieux de culture, et les cellules sont ensuite incubées à 36.5°C / 5% CO2 pendant 24 heures pour le test de cytotoxicité et 5 jours pour le dosage de mélanine.

a) Application des solutions à tester sur les mélanocytes : Les concentrations à tester sont mises en contact avec les mélanocytes en confluence dans les plaques 96 (test de cytotoxicité) et 24 (dosage de mélanine) puits. Pour chaque concentration le test est réalisé sur 3 puits. Les plaques sont incubées pendant 24 $\pm$ 1 heures et 5 jours à 36.5°C / 5% CO2.

b) Test de viabilité : La viabilité cellulaire est évaluée par la méthode MTT sur les cellules après 24 heures d'incubation avec les produits. Après 24 heures d'incubation, les puits prévus sont rincés 1 fois avec 200 $\mu$L de PBS. Dans chaque puits 50 $\mu$l de solution de MTT à 0,5 mg/ml sont ajoutés et l'incubation est effectuée pendant 3 heures à 36.5°C / 5% CO2. Dans chaque puits 150 $\mu$l d'isopropanol sont ajoutés. Après homogénéisation, une lecture de l'absorbance est réalisée à 550 nm. Pour chaque condition, le rapport de la moyenne des densités optiques des cellules sur la moyenne des densités optiques des contrôles négatifs déterminera le taux de viabilité.

Une valeur seuil de viabilité à 70 % par rapport à la valeur du contrôle négatif est utilisée pour classifier les substances testées comme cytotoxiques ou non cytotoxiques. Une classification « non cytotoxique » est donnée sur les résultats *in vitro* pour une viabilité > 70% et une classification « cytotoxique » pour une viabilité $\leq$ 70%.

La concentration à 5% de l'extrait selon l'invention s'est montrée cytotoxique à 5% dans les conditions du test. Les concentrations 2%, 1% et 0.1% sont donc utilisées pour le test de modulation de la mélanine. La quantité de mélanine présente dans les cellules est dosée après la lyse cellulaire. Les résultats sur l'inhibition de la production de mélanine sont donnés ci-dessous.

[Tableaux9]

|  | Extrait concentration | Croissance cellulaire versus contrôle (%) | Inhibition Mélanine versus contrôle (%) | Contenu en Mélanine ($\mu g/ml$) |
|---|---|---|---|---|
| Extrait *peregrina* selon l'invention | 2% | -3.41 | +16.50 | 152 |
|  | 1.0% | +13.46 | +65.90 | 62 |
|  | 0.10% | +13.29 | +71.40 | 52 |

Conclusion : l'extrait *peregrina* selon l'invention inhibe la production de mélanine *in cellulo* ce qui lui confère une propriété protectrice de la peau. Cette inhibition montre également un effet relaxant sur les mélanocytes car le taux basal est inférieur au témoin (absence d'extrait selon l'invention dans le milieu de culture) ; il est rappelé que la production de mélanine est une réponse à un stress cellulaire. Ainsi, l'extrait *peregrina* selon l'invention démontre pouvoir prévenir les tâches du vieillissement.

Exemple 10: Caractérisation analytique de l'extrait *peregrina* selon l'invention versus l'extrait *oleifera* avec un procédé d'extraction identique

Sur la base du tourteau de *Moringa peregrina* et de celui de *Moringa oleifera,* le procédé d'extraction selon l'invention décrit à l'exemple 1 a été appliqué. Il est donné ci-après la composition comparative des ingrédients extraits sur la base de la matière sèche.

[Tableaux 10]

| Composés | Oleifera (%) | Peregrina (%) |
|---|---|---|
| Acide acétique | 1.431 | - |
| 1-hydroxy-2-propanone | 1.962 | - |
| Furfural | 6.140 | 2.528 |
| 2,5-Furandicarboxaldéhyde (DFF) | 0.823 | 55.660 |
| Myristate d'isopropyle | 0.128 | 1.175 |
| Palmitate de méthyle | 0.230 | - |
| Acide palmitique | 10.356 | 4.713 |
| Palmitate d'éthyle | 0.399 | - |
| Linoléate de méthyle | 0.428 | - |
| Oléate de méthyle | 2.443 | - |
| Acide oléique | 47.844 | 11.093 |
| Oléate d'éthyle | 2.675 | - |
| Acide stéarique | 5.064 | - |
| Hexanedioate de bis 2-éthyl hexyle | 1.688 | - |
| 7-oxo-déhydroabiétate de méthyle | 0.327 | - |
| 1,4-téréphtalate de 2-ethyl-hexyle | 0.655 | - |
| Total | 84.317 | 74.169 |

Il apparaît que les deux extraits présentent un profil moléculaire très différent. L'extrait de *Moringa oleifera* contient moins de 1% de DFF alors que l'extrait de *Moringa peregrina* en contient plus de 50%.

Exemple 11: Test comparatif avec *Moringa oleifera* pour test *in tubo* collagénase

L'objectif de cette étude est d'évaluer la modulation de l'activité inhibitrice des Métalloprotéases par l'extrait *peregrina* selon l'invention dans un modèle *in vitro* acellulaire faisant appel à une collagénase de type I et une hyaluronidase, un complexe de substrat et un chromophore, Ninhydrine. Une solution tamponnée de Collagénase de type I et hyaluronidase réagit avec un complexe de substrat spécifique et le transforme pour former un composé capable d'activer un chromophore par incubation à 80°C pendant 15 mn. Les activités de la collagénase et de la hyaluronidase peuvent ainsi être évaluées par mesure de l'absorbance à 565 nm. L'échantillon est mis en contact avec la solution de Collagénase et Hyaluronidase en même temps que le complexe de substrat des enzymes à 37°C pendant 5 minutes. Le substrat transformé par les enzymes est capable d'activer le chromophore par incubation à 80°C pendant 15 minutes. L'activité de la Collagénase et Hyaluronidase en présence/absence de l'échantillon est alors évaluée par mesure de l'absorbance à 565 nm. La modulation de cette activité est exprimée en pourcentage d'inhibition ou d'activation de l'activité de la Collagénase et Hyaluronidase en absence d'actif, c'est-à-dire uniquement en présence du substrat de l'enzyme.
Protocole : Une solution d'enzymes Collagénase de type I et Hyaluronidase est incubée dans son substrat, pendant 5 minutes, en absence ou présence de l'extrait *peregrina* selon l'invention testé. Les solutions sont ensuite mises en contact avec le chromogène, Ninhydrin, avant une incubation de 15 minutes à 80°C. A la fin de la période d'incubation, l'activité des enzymes Collagénase et Hyaluronidase avec et sans produit à l'essai ou de référence a été évaluée par mesure de l'absorbance des milieux réactionnels à 565 nm. Pour chaque concentration testée, la modulation de l'activité des enzymes Collagénase et Hyaluronidase par le produit à l'essai est calculée selon la formule suivante.

[Math. 6]

Pourcentage de modulation de l'activité des enzymes Collagénase/ Hyaluronidase =

100 x [(DO produit à l'essai ou de référence – DO Collagénase/Hyaluronidase

seule)/DO Collagénase/Hyaluronidase seule].

Si le résultat est négatif, le pourcentage est exprimé en inhibition des enzymes ; si le résultat est positif, le pourcentage est exprimé en activation des enzymes. Le résultat de l'inhibition des Métalloprotéases est donné ci-dessous.

[Tableaux11]

| | | Inhibition versus Contrôle (%) |
|---|---|---|
| Extrait *peregrina* selon l'invention | 1% | 87 |
| | 0.5% | 87 |
| | 0.1% | 88 |
| | 0.01% | 62 |

Conclusion : L'extrait *peregrina* selon l'invention engendre une forte inhibition des Métalloprotéases (Collagénase / Hyaluronidase). Il est capable d'inhiber à 88% ces Métalloprotéases dès la concentration de 0.1% et a un bon potentiel pour protéger avec une grande efficacité la matrice extra-cellulaire de la peau et, par cette inhibition, il montre un effet anti-vieillissement.

A comparer avec l'extrait selon le brevet FR2946879 de Pierre Fabre dont voici les résultats selon le même test.

[Tableaux12]

| | | Inhibition versus Contrôle (%) |
|---|---|---|
| Extrait *Moringa oleifera* selon Brevet de Pierre Fabre | 1% | Concentration non compatible avec système d'essai |
| | 0.5% | 4 |
| | 0.1% | 24 |
| | 0.01% | 42 |

Conclusion l'extrait selon le brevet de Pierre Fabre montre une légère action inhibitrice sur l'activité collagénase en dose-dépendance inverse avec un pic d'inhibition de 42% toutes concentrations confondues contre un pic d'inhibition de 100% pour l'extrait *peregrina* selon l'invention.

L'activité anti-âge sur ce paramètre apparaît différente et nouvelle comparativement aux effets observés avec l'extrait selon le brevet Pierre Fabre.

Exemple 12: Tests comparatifs pour l'activité anti-stress (par inhibition de la PLA2).

Pour l'activité anti-stress par inhibition de la PLA2 sur la peau qui apporte une orientation apaisante/anti-stress à effet anti-âge, deux tests in tubo PLA2 additionnels ont été menés; un avec le procédé selon l'invention réalisé sur le tourteau d'oleifera (extrait fait à l'identique que celui sur *peregrina* dans le procédé selon l'invention), un avec l'extrait correspondant au document FR2946879 de Pierre Fabre, un autre encore correspondant au brevet FR3076460 de BASF Beauty Care Solutions avec le produit Purisoft® et enfin un dernier avec le document FR2825267 de Chuun & Thurot.

L'objectif de cette étude est d'évaluer la modulation de l'activité anti-inflammatoire de l'enzyme phospholipase A2 par un ou plusieurs échantillons dans un modèle In Vitro acellulaire faisant appel au kit d'analyse « SPLA2 (type V) Inhibitor Screening Assay Kit ».

Une solution tamponnée de Phospholipase A2 réagit avec un substrat spécifique, le diheptanoyl thio-PC, et le transforme pour former un composé qui se lie à un chromogène, le DTNB sous agitation à température ambiante. L'activité de la phospholipase A2 peut ainsi être évaluée par mesure de l'absorbance à 413 nm.

Les produits « Extrait *peregrina* selon l'invention » ou le produit de référence inhibiteur « Thioetheramide-PC » sont mis en contact avec la solution de Phospholipase A2 en même temps que le substrat de l'enzyme. Le substrat transformé par l'enzyme est coloré à l'aide du chromogène DTNB par agitation à température ambiante. L'activité de la Phospholipase A2 en présence/absence du produit « Extrait *peregrina* selon l'invention» ou du produit de référence est alors évaluée par mesure de l'absorbance à 413 nm.

La modulation de cette activité est exprimée en pourcentage d'inhibition ou d'activation de l'activité de la Phospholipase A2 en absence d'actif, c'est-à-dire uniquement en présence du substrat de l'enzyme (diheptanoyl thio-PC).

L'inhibiteur « Thioetheramide-PC » à la concentration de 1mg dans 100$\mu$l est le produit de référence (Témoin actif) dans cette étude ce dernier inhibe à 93% l'activité de la PLA2 ce qui valide le test.

Une solution d'enzyme Phospholipase A2 est incubée dans son substrat, diheptanoyl thio-PC, en absence ou présence de l'inhibiteur de référence et du produit « Extrait *peregrina* selon l'invention» testé puis le chromogène DTNB est incorporé avant une incubation de 15 minutes à 25°C.

A la fin de la période d'incubation, l'activité de l'enzyme Phospholipase A2 avec et sans produit à l'essai ou de référence a été évaluée par mesure de l'absorbance des milieux réactionnels à 413 nm. Pour chaque concentration testée, la modulation de l'activité de l'enzyme Phospholipase A2 par le produit à l'essai est calculée selon la formule suivante:

[Math. 7]

Pourcentage de modulation de l'activité de l'enzyme Phospholipase A2 = 100 x [(DO405 produit à l'essai ou de référence – DO405 sPLA2 seule) /DO405 sPLA2 seule]

Si le résultat est négatif, le pourcentage est exprimé en inhibition de l'enzyme ; si le résultat est positif, le pourcentage est exprimé en activation de l'enzyme.

[Tableaux13]

| Extrait *peregrina* selon l'invention | 1% | 19 |
|---|---|---|
| | 0.1% | 16 |
| | 0.01% | 11 |

La constance et l'activité sensiblement dose-dépendante de cet extrait *peregrina* selon l'invention sur l'inhibition de la PLA2 mettent en lumière une activité apaisante par réduction de l'intensité en amont de la cascade arachidonique. Une telle action apaisante très en amont de la cascade arachidonique réduit l'impact du stress physiologique basal. L'extrait *peregrina* selon l'invention est par conséquent un anti-stress.

Avec l'extrait protocole issu du brevet FR2946879 de Pierre Fabre, on obtient les résultats suivants.

[Tableaux14]

| Extrait de Pierre Fabre | 1% | 0 |
|---|---|---|
| | 0.1% | 16 |
| | 0.01% | 8 |

À 1%, ce qui correspond à son dosage d'emploi minimum, l'extrait ne montre pas d'activité. A la dilution, une activité apparaît mais le manque d'effet dose-dépendante ne permet pas de valider une action spécifique et fiable de l'extrait sur l'inhibition de cette enzyme.

Avec l'extrait protocole issu du brevet FR3076460 de BASF Beauty Care Solutions avec le produit Purisoft®

LS9726, on obtient les résultats suivants.

[Tableaux15]

| Extrait BASF Purisoft LS9726 | 1% | 10 |
|---|---|---|
| | 0.1% | 12 |
| | 0.01% | 6 |

Cet extrait montre une légère action inhibitrice qui n'atteint pas le maximum observé à 1% avec l'extrait *peregrina* selon l'invention, à savoir 19 % d'inhibition pour l'extrait selon l'invention contre 10% d'inhibition à 1% pour cet extrait.

Avec l'extrait protocole issu du brevet FR2825267 de Chuun & Thurot nous observons les résultats qui suivent.

[Tableaux16]

| Extrait de Chuun & Thurot | 1% | 0 |
|---|---|---|
| | 0.1% | 0 |
| | 0.01% | 0 |

Cet extrait ne montre pas d'action inhibitrice sur cette enzyme.

Avec l'extrait protocole issu du procédé selon la présente invention mais appliqué au tourteau de *Moringa oleifera* en lieu et place du tourteau de *Moringa peregrina,* nous observons les résultats qui suivent.

[Tableaux17]

| Extrait *Moringa oleifera* | 1% | Non dosable |
|---|---|---|
| | 0.1% | 0 |
| | 0.01% | 6 |

Cet extrait ne montre pas d'action inhibitrice significative ou stable sur cette enzyme. Conclusion : seul l'extrait *peregrina* selon l'invention démontre une activité inhibitrice significative sur l'enzyme PLA2.

Exemple 13: Formulation d'un produit maquillant

[Tableaux18]

| Ingrédients | % |
|---|---|
| Eau | QSP |
| Caprylic / Capric tri-glyceride | 19.0000 |
| Gomme Acacia sénégal | 7.0000 |
| Charcoal | 6.0000 |
| Glycerine | 5.0000 |
| Propanediol | 5,0000 |
| Bentonite | 3.1500 |
| Cetearyl glucoside | 3.0000 |
| Cetearyl alcohol | 3.0000 |
| Benzyl alcohol | 1.0000 |
| Extrait *peregrina* selon invention | 2.0000 |
| Gomme Cellulose | 0.8000 |

(suite)

| Ingrédients | % |
|---|---|
| Gomme de Xanthane | 0.1750 |
| Acide Citrique | 0.1750 |

Exemple 14 : Formulation d'un produit lavant

[Tableaux19]

| Ingrédients | % |
|---|---|
| Eau | QSP |
| Sodium coco-sulfate | 5.0000 |
| Sodium cocoyl isethionate | 4.0000 |
| Bentonite | 3.7800 |
| Caprylic / Capric tri-glyceride | 2.0000 |
| Extrait *peregrina* selon l'invention | 1.0000 |
| Gluconolactone | 0.7500 |
| Sodium benzoate | 0.5450 |
| Parfum | 0.5000 |
| Gomme de Xanthane | 0.2700 |
| Sodium stearoyl glutamate | 0.2250 |
| Acide Citrique | 0.2250 |
| Calcium gluconate | 0.0050 |

Exemple 15 : formulation d'un produit de soin (crème anti-stress anti-âge)

[Tableaux20]

| Ingrédient | % |
|---|---|
| Eau | QSP |
| Caprylic / Capric tri-glyceride | 18.0000 |
| Bentonite | 4.2000 |
| Cetearyl alcohol | 1.5000 |
| Extrait *peregrina* selon invention | 5.0000 |
| Gluconolactone | 0.7500 |
| Sodium benzoate | 0.5450 |
| Gomme de Xanthane | 0.5000 |
| Parfum | 0.5000 |
| Sodium stearoyl glutamate | 0.2500 |
| Acide Citrique | 0.2500 |
| Calcium gluconate | 0.0050 |

Exemple 16 : formulation par voie orale pour nutricosmétique

Un comprimé de 1 g pour une activité apaisant/ anti-stress comprend : 3% extrait sec selon l'invention (contenant 0.6% de 2,5-diformylfuran sur un support d'inuline) + 47% de carbonate de calcium contenant 200 UI de vitamine D + 25% gluconate de magnésium + 22% inuline + 3% stéarate de magnésium.

Exemple 17 : Tests toxicologiques de l'extrait *peregrina* selon l'invention

Préparation de l'extrait *peregrina* conformément à l'exemple 1 :
Des graines de *Moringa peregrina* (Forssk.) Fiori, récoltées à maturité du fruit, ont été séchées pour obtenir un taux d'humidité interne autour de 6%, puis pressées avec une presse mécanique à vis sans fin, de sorte à séparer l'huile du reste de la graine pour obtenir d'une part l'huile vierge et d'autre part un tourteau. Le tourteau est alors isolé sous forme de boudins prédécoupés en morceau de 1 à 2 cm. A partir du tourteau on effectue une macération et extraction avec de l'éthanol à 96° préchauffé 10 mn à 55°C dans le ratio 25/75% (m/m), On cisaille au mixer pendant 15 mn puis on laisse agiter à l'hélice pendant 2 h à 20°C. On filtre alors au Büchner sous vide et on obtient un filtrat jaune pâle contenant 1.15% de matière sèche. Ce filtrat est utilisé pur dans les tests qui suivent.

Détermination de l'activité mutagène sur la souche bactérienne *Salmonella typhimurium* (TA 100) - Test de mutation bactérienne inverse

Le test s'est déroulé en 3 *phases* principales :

- Une expérience préliminaire est réalisée afin d'évaluer la cytotoxicité de l'élément à tester et de sélectionner la gamme de doses pour les expériences ultérieures,
- Une première expérience de génotoxicité (Test 1), avec et sans activation métabolique, avec incorporation directe du système de test et du test (ou des contrôles) sur gélose minimale, sur la plage de doses définie par l'étude préliminaire,
- Une deuxième expérience (Test 2), avec pré-incubation du système de test et de l'élément de test (ou des contrôles), avec et sans activation métabolique, avec des niveaux de dose définis par le responsable de l'étude après analyse des résultats de la première expérience. Cette seconde expérience a été réalisée afin de confirmer ou de compléter les résultats de la première, en particulier lorsque des résultats équivoques ou négatifs ont été obtenus.

Les dilutions des éléments de test ont été préparées dans l'eau pour analyse.

Le test de cytotoxicité a été réalisé sur la souche S. *typhimurium* TA100 aux concentrations de 5000, 1600, 500, 160 et 50 $\mu$g / plaque, avec et sans S9-Mix.

Les réactifs utilisés pour la préparation de S9-Mix ont été préparés selon les instructions suivantes :

[Tableaux21]

|  | Concentration finale |
|---|---|
| MgCl2 (0.4 M) + KCl (1.65 M) | 8 mM + 33 mM |
| Glucose 6 Phosphate (0.2 M) | 5 mM |
| NADP (0.1 M) | 4 mM |
| Tampon Phosphate pour S9-Mix (pH 7.4 - 0.2 M) | 0.1 M |
| S9 fraction | 10% |
| Eau | Ajuster pour concentration finale |

Les bactéries ont été exposées à l'extrait de test avec et sans système d'activation métabolique. Le système métabolique utilisé est une fraction post-mitochondriale améliorée par cofacteur (S9). Cette fraction S9, une fraction microsomale d'homogénat de foie de rat Sprague Dawley traité avec un inducteur enzymatique, est préparée selon Maron, D.M. et Ames, B.N. (1983) et a été fournie par MOLTOX TM. Elle est conservée à une température inférieure à -70 ° C. La fraction microsomale S9 a été utilisée à la concentration de 10% dans S9-Mix. Le protocole appliqué a été le suivant :

- Dans 3 tubes d'hémolyse, a été introduit :

o dosage sans activation métabolique:

- 0,1 ml des différentes concentrations des éléments de test,
- 0,5 ml de tampon phosphate stérile 0,2 M, pH 7,4,
- 2 ml de top agar pour S. *typhimurium,*
- 0,1 ml d'inoculum bactérien (TA100).

◦ dosage avec activation métabolique:

- 0,1 ml des différentes concentrations des éléments de test,
- 2 ml de top agar pour S. *typhimurium,*
- 0,1 ml d'inoculum bactérien (TA100),
- 0,5 ml de S9-Mix.
- Mélanger et verser sur la surface de la gélose de fond préalablement répartie dans des boîtes de Pétri.
- Incuber à 37 ° C ± 2 ° C pendant 48 à 72 heures.

Ces dosages ont été réalisés pour chaque test : test préliminaire de cytotoxicité, test 1 et test 2. Le contrôle non traité, les contrôles négatifs et les contrôles positifs réalisés au cours de la méthode de pré-incubation ont été incubés pendant 20 à 30 minutes à 37 ° C ± 2 ° C avant de verser la gélose supérieure.

Le protocole appliqué a été le suivant :

- Dans 4 fractions de 2 ml d'agar top pour *S. typhimurium,* introduire :

  ◦ 0.1 ml de tampon phosphate 0,2 M, pH 7,4,
  ◦ 0.1 ml de solvant,
  o 0.1 ml de S9-Mix.
  ◦ 0.1 ml de la préparation de l'élément à tester à la concentration la plus élevée,

- Une fraction de 2 ml d'agar top pour *S. typhimurium* est utilisée pour contrôler sa stérilité.
- Mélanger et verser sur la surface de la gélose de fond préalablement répartie dans des boîtes de Pétri.
- Incuber à 37 ° C ± 2 ° C pendant 48 à 72 heures.
- Le test est réalisé en triple.
- Aucune croissance bactérienne ne doit être observée.

Pour au moins 5 concentrations de l'extrait à tester, un test sans activation métabolique et un test avec activation métabolique ont été réalisés.

Expression des résultats et interprétation

De nombreux critères permettent de déterminer si un résultat est positif, notamment une augmentation du nombre de révertants corrélée à la dose d'item à tester, ou une augmentation reproductible du nombre de révertants à une ou plusieurs concentrations, avec ou sans activation métabolique.

- L'élément à tester est considéré comme mutagène si à l'issue des étapes de vérification, il a été obtenu, de manière reproductible, une relation dose-effet sur une ou certaines des 5 souches avec et / ou sans activation métabolique. La mutagénicité n'est prise en compte pour une concentration donnée que lorsque le nombre de révertants est au moins égal au double du taux de réversion spontanée pour les souches TA98, TA100 et TA102 ($R \geq 2$) et au triple du taux spontané de réversion pour les souches TA1535 et TA1537 ($R \geq 3$).
- L'élément de test est considéré comme non mutagène si, à l'issue du test 1 et du test 2, le taux de révertants est toujours resté inférieur au double du taux de réversion spontanée pour toutes les concentrations de l'élément de test testé, pour les souches TA98, TA100 et TA102 ($R < 2$) et inférieur au triple du taux de réversion spontanée pour les souches TA1535 et TA1537 ($R < 3$), avec et sans activation métabolique, et à condition qu'il ait été vérifié que l'absence de l'effet mutagène n'est pas liée à la toxicité des concentrations testées.

L'étude préliminaire n'a montré aucune cytotoxicité de l'élément de test ; par conséquent, cette gamme de concentrations a été utilisée pour le test de génotoxicité 1.
En fonction du résultat obtenu pour le test 1, il a été décidé d'utiliser la même gamme de dilution pour le test 2. L'analyse des révertants montre que:

- Aucun effet cytotoxique n'a été observé,
- Aucune concentration de l'extrait à tester n'a montré un rapport R supérieur ou égal au moins au double du taux de réversion spontanée pour TA98, TA100 et TA102 et au triple du taux de réversion spontanée pour TA1535 et TA1537, avec et sans activation métabolique,
- Aucune réponse à la dose n'a été observée, quels que soient le système de test ou les conditions du test.

Au vu des résultats obtenus au cours de cette étude, l'extrait *peregrina* selon l'exemple 1 peut être considéré comme ne présentant aucune activité mutagène ni pro-mutagène.

Test de photo-toxicité *in vitro* 3T3 NRU

Le principe du test a pour base la comparaison de la cytotoxicité de l'extrait *peregrina* selon l'exemple 1 en présence et en absence d'une dose non cytotoxique d'UVA, sur des cellules en culture. La cytotoxicité est évaluée par la détermination de la viabilité cellulaire à l'aide d'un colorant vital : le rouge neutre, 24h après le traitement avec les éléments de référence et l'extrait de *M. peregrina* avec ou sans irradiation aux UVA. Les cellules utilisées sont des fibroblastes d'embryon de souris de lignée Balb/c 3T3 clone 31 (ATCC - CCL163). Le contrôle positif est une solution de chlorpromazine (Numéro CAS : 69-09-0). Le contrôle négatif est un diluant de l'extrait testé et de référence (solution saline tamponnée +/- 1 % solvant). L'extrait *peregrina* a été testé à 8 concentrations sur au moins quatre puits de culture par concentration étudiée, en présence ou en absence d'UVA. Les fibroblastes ont été trypsinés et deux plaques de culture 96 puits ont été ensemencées à raison de 100 $\mu$l d'une suspension cellulaire à $2.10^5$ cellules/ml (soit $2.10^6$ cellules par puits) dans du milieu de culture complet.

Les plaques ensemencées ont été incubées à l'étuve 24 heures à 37°C, 5% $CO_2$. A la fin de l'incubation, la semi-confluence du tapis cellulaire a été vérifiée. Les dilutions ont été préparées juste avant leur dépôt sur les cellules. Le pH de la plus forte concentration a été mesuré ; il est compris entre 6.5 et 8. Le milieu de culture a été éliminé, chaque puits a été préalablement rincé délicatement avec 150 $\mu$l de PBS maintenu à température ambiante avant d'être traité avec 100 $\mu$l de chaque dilution de l'extrait ou de référence. Les plaques de cultures ont été incubées à l'obscurité durant 1h plus ou moins 5 minutes à 37°C et 5 % de $CO_2$. L'irradiation a été réalisée à l'aide d'un irradiateur solaire BIO SUN (Vilber Lourmat RMX3W). Le BIO SUN est un système qui contrôle l'irradiation UV à l'aide d'un microprocesseur programmable. Le système suit en continu l'émission de lumière UV. L'irradiation s'arrête automatiquement quand l'énergie délivrée est égale à l'énergie programmée. L'irradiation spectrale du dispositif à l'essai a été mesurée dans la gamme de longueurs d'onde 250 à 700 nanomètre avec un spectroradiomètre calibré. Une des 2 plaques a été irradiée avec son couvercle à température ambiante, et l'autre plaque a été protégée des UVA et conservée à température ambiante le temps de l'irradiation. Après l'irradiation, le milieu de traitement a été aspiré et les cellules ont été rincée. Puis sans $\mu$l de milieu de culture complet ont été ajoutés doucement et les plaques ont été incubées pendant 18 à 22h à 37°C et 5 % de $CO_2$. Le lendemain la viabilité cellulaire (croissance, morphologie, intégrité de la monocouche) a été évaluée par observations sur un microscope à contraste de phase. Le milieu de culture a été éliminé, chaque puits a été préalablement rincé et maintenu à température ambiante avant d'être traité avec 100 $\mu$l de la solution de coloration. Les plaques ont été remises à l'incubateur durant 3h dans les mêmes conditions. La solution colorante a été éliminée et les cellules ont été rincées, puis la solution de rinçage a été enlevée et 150 $\mu$l de solution de désorption ont été ajoutés dans chaque puits. Les plaques ont été agitées jusqu'à la solubilisation complète des cristaux. Les absorbances ont été mesurées à 450 nm.

Validation du test :

La sensibilité des cellules aux UVA est contrôlée tous les 10 passages environ, par évaluation de leur viabilité après exposition à des doses croissantes d'irradiation. Les cellules sont mises en culture à la densité utilisée dans l'essai. Elles sont irradiées le jour suivant à la dose de 2,5 et 9 J/cm$^2$ et la viabilité cellulaire est déterminée un jour plus tard à l'aide du test NRU. Les cellules répondent aux critères de qualité si leur viabilité après irradiation à 5 J/cm2 UVA est supérieur ou égal à 80 % de la viabilité des témoins maintenus à l'obscurité : à la dose la plus forte de 9 J/cm2 UVA, la viabilité doit être au moins égal à 50% de celle des témoins maintenus à l'obscurité.

Résultats :

Le contrôle négatif a une absorbance supérieure ou égale à 0.4. La chlorpromazine, contrôle positif, présente une CI$_{50}$ comprise entre 0.1 et 2 $\mu$g/ml en présence d'UVA et comprise entre 7 et 90 $\mu$g/ml en absence d'UVA. Ces résultats permettent de valider le test. La concentration de l'extrait de tourteau de *peregrina* donnant 50% de mort cellulaire en présence ou en absence d'UVA ne peut être estimée. La mortalité n'a jamais atteint 50%. La concentration de l'extrait de tourteau de *peregrina* donnant 50% de viabilité cellulaire en présence ou en absence d'UVA ne peut être estimée. La viabilité est toujours supérieure à 50%.

Conclusion : Dans les conditions expérimentales retenues l'extrait de tourteau de *peregrina* peut être considéré comme non phototoxique.

_Evaluation du potentiel irritant oculaire par l'étude de la cytotoxicité in vitro selon la méthode de relargage du rouge neutre sur lignée cellulaire SIRC_

Cette étude in vitro a pour base l'évaluation de la cytotoxicité de l'extrait de tourteau de *peregrina* par détermination de la concentration entraînant 50% de mortalité cellulaire ($CI_{50}$) sur une monocouche cellulaire à l'aide de la technique de relargage du rouge neutre. Les cellules utilisées sont des fibroblastes de cornée de lapin SIRC (ATCC - CCL60) exemptes de mycoplasmes.

L'extrait *peregrina* a été dilué dans du sérum physiologique à 25% et 50%. Les fibroblastes ont été trypsinés et deux plaques de culture 24 puits ont été ensemencées à raison de 1 ml d'une suspension cellulaire à $2.10^5$ cellules/ml dans du milieu de culture complet. Les plaques ensemencées ont été incubées à l'étuve une nuit à 37°C, 5% $CO_2$. A la fin de l'incubation, la confluence du tapis cellulaire a été vérifiée. La solution de coloration a été préparée à 0,5 mg/ml dans du milieu de culture complet. Le milieu de culture a été éliminé, 1 ml de la solution colorante a été déposée dans chaque puits. Les plaques ont été remises à l'incubateur à 37°C, 5% CO2 pendant 3h +/- 15 minutes. Après ce temps de contact, la solution colorante a été éliminée et remplacée par 1 ml de milieu de culture complet par puits. Les plaques ont été maintenues à température ambiante pendant au moins 30 minutes afin de stabiliser le système avant le contact avec l'extrait ou la référence. Chaque puits a été rincé avec 2 ml de PBS, maintenu à température ambiante, puis 500 μl de chaque dilution d'extrait *peregrina* ou de référence ont été déposées au contact du tapis cellulaire. Le temps de contact a été de 60 secondes (30 secondes pour le contrôle positif). Le traitement a été réalisé puits par puits avec déclenchement du chronomètre au moment du dépôt de l'extrait *peregrina* ou la référence. La plaque a été agitée manuellement pendant toute la durée du traitement. Après 55 secondes (ou 25 secondes pour le contrôle positif, la dilution a été aspirée. A 60 ou 30 secondes précise, 5 rinçages successifs ont été effectués (5 x 2 ml de PBS maintenus à température ambiante). Le surnageant a été aspiré après chaque rinçage et après le dernier rinçage les puits sont restés dépourvu de milieu en attendant la phase de révélation. Après traitement complet de la plaque de culture, 1 ml de la solution de désorption a été déposée dans chaque puits. La plaque est agitée pendant environ 15 minutes jusqu' à l'obtention d'une coloration homogène. Les solutions obtenues pour chaque puits de culture ont été prélevées et réparties dans 2 puits d'une plaque 96 puits, soit 150 μl/puits.

Résultats :

La concentration de l'extrait *peregrina* donnant 50% de mort cellulaire a été évaluée à >50%. Le pourcentage de mort cellulaire à 50% d'extrait *peregrina* a été évalué à 17%

Conclusion : Dans les conditions expérimentales retenues, la cytotoxicité de l'extrait de tourteau de *peregrina* peut être classée comme cytotoxicité négligeable.

_Evaluation de la compatibilité cutanée d'un extrait peregrina après application unique sous pansement occlusif pendant 48 heures sous contrôle dermatologique._

Le but de cette étude est d'évaluer le degré de compatibilité cutanée de l'extrait *peregrina* par test épicutané, réalisé au niveau de la face antéro externe du bras pendant 48 heures ; et d'une manière générale évaluer la capacité de l'extrait *peregrina* à maintenir la peau en bon état. 10 volontaires saints, féminin ou masculin âgés de 18 à 65 ans, n'ayant ni la peau sèche ni la peau sensible et indemnes de toute lésion dermatologique sur la zone de traitement devaient être inclus dans l'étude. La compatibilité cutanée de l'extrait *peregrina,* préparé sous forme de lotion avec 5% de l'extrait *peregrina* selon l'exemple 1 et 95% d'un mélange Propanediol/Sorbitol, a été évaluée 48 heures après l'application initiale entre 30 à 40 minutes après le retrait du pansement. Le comptage des réactions cutanées (érythème et oedème) a été effectuée de 0 à 3 selon les échelles suivantes :

[Tableaux22]

| Cotation | Erythème (E) | OEdème (Oe) |
|---|---|---|
| 0 | aucun érythème | aucun œdème |
| 0,5 | Érythème à peine perceptible, coloration très légèrement rosée sur une partie de la zone de patchage | œdème palpable à peine perceptible |

(suite)

| Cotation | Erythème (E) | OEdème (Oe) |
|---|---|---|
| 1 | Érythème léger, coloration rosée sur toute la surface de patchage | œdème palpable et visible |
| 2 | Érythème modéré, coloration nette sur toute la surface de patchage | œdème net avec ou sans papules ou vésicules |
| 3 | Érythème important, coloration intense sur toute la zone de patchage | œdème important diffusant en dehors de la zone de patchage avec ou sans papules ou vésicules |

Toute autre réaction cutanée (bulles, papules, vésicules, sécheresse, desquamation, rugosité, effet savon...) a été évaluée selon l'échelle suivante et rapportée de manière descriptive :

- 0 : aucune réaction,
- 0,5 : très léger
- 1 : léger
- 2 : modéré
- 3 : important.

A la fin de l'étude, un indice d'irritation moyen (I.I.M.) a été calculé selon la formule suivante :

[Math. 8]

I.I.M.= Somme des réactions cutanées (E + Oe + bulles + papules + vésicules)/ Nombre de volontaires analysés

L'I.I.M. obtenu a permis de classer l'extrait testé selon le barème présenté dans le tableau ci-après :

I.I.M. $\leq$ 0,20 Non irritant
0,20 < I.I.M. $\leq$ 0,50 Légèrement irritant
0,50 < I.I.M. $\leq$ 2 Moyennement irritant
2 < I.I.M. $\leq$ 3 Très irritant

Résultats : L'Indice d'Irritation Moyen (I.I.M.) de l'extrait de tourteau de *peregrina* est égal à : 0.
Conclusion : L'extrait de tourteau de *peregrina* peut être considéré comme non irritant après 48 heures consécutives d'application chez 12 volontaires.

Conclusion générale des tests :

Les résultats des tests effectués ci-dessus sont concluants et démontrent pour l'extrait *peregrina* selon l'exemple 1:

1. Les tests d'irritation oculaire et cutanée sont négatifs
2. Les tests de phototoxicité sont négatifs
3. Les tests de mutagénicité sont négatifs.

La sécurité de l'extrait *peregrina* selon l'invention est démontrée et idéale pour une utilisation cosmétique topique à grande échelle sans restriction sur les populations cibles.

Exemple 18 : Évaluation par mesure de la Perte Insensible en Eau (PIE) sur la barrière cutanée et de son acceptabilité après utilisation sur une période de 21 jours

Le produit étudié dans cette étude est le produit de soin sous forme de crème de l'exemple 15. Ce produit est appliqué matin et soir sur le visage propre en massant délicatement en évitant le contour des yeux. Les mesures sont effectuées au niveau des joues.

Les critères d'évaluation sont:

- évaluation de l'effet sur la barrière cutanée : comparaison des valeurs de PIE avant toute application du produit (J1) puis après 21 jours d'application (J21).
- remontée des sensations d'inconfort éventuelles à J21.
- acceptabilité cosmétique : questionnaire complété par le volontaire à J21. 22 femmes volontaires âgées de 50 ans en moyenne (entre 20 et 70 ans) avec tout type de peau ont été testées. Le produit évalué sur la barrière cutanée a donné les résultats suivants (*=% de variation J21 par rapport à J1, **=Test de Wilcoxon pour données appariées avec S= significatif (p≤0,05 et NS=non significatif (p>0,05) :

[Tableaux23]

|  | Valeur de PIE ($g/m^2.h$) | |
|---|---|---|
|  | J1 | J21 |
| Moyenne | 11.70 | 11.79 |
| Ecart type | 3.26 | 2.87 |
| Médiane | 10.39 | 11.40 |
| Minimum | 7.97 | 7.12 |
| Maximum | 17.30 | 17.14 |
| % de variation* | - | 0.83 |
| Valeur de p** Significativité | - | p=0.555 (NS) |

L'analyse des résultats démontre que la PIE est restée stable à J21 comparativement à J1 : le produit a présenté un effet « dermo-protecteur » après 21 jours d'application. Compte tenu de l'absence de baisse significative des valeurs de PIE à J21 comparativement à J1, l'effet « nourrissant » du produit testé n'a pas pu être mis en évidence par mesures instrumentales. 81 % des volontaires ont répondu positivement à la question « la peau est nourrie » dans le questionnaire d'acceptabilité à J21.

Conclusion : Dans les conditions de l'étude, la crème a présenté un effet « dermo-protecteur », mis en évidence par mesure de PIE et une bonne acceptabilité cosmétique, avec 86 % d'opinions favorables.

**Revendications**

1. Extrait de graines de *Moringa peregrina,* **caractérisé en ce qu'**il est obtenu par extraction solide-liquide du tourteau des graines non décortiquées, sous agitation, dans une proportion d'environ 25% en poids de matière solide par rapport au poids total mis en oeuvre dans un solvant majoritairement alcoolique, l'alcool étant choisi parmi l'éthanol ou le méthanol avec éventuellement un co-solvant de type polyol ou eau subcritique, dans une proportion de 70 à 100% en poids d'alcool par rapport au poids total du solvant, à une température comprise entre 16 et 30°C pendant une durée d'environ 2 heures et par séparation des phases liquide et solide de manière à éliminer la phase solide et récupérer un extrait liquide de graine de *Moringa peregrina,* ledit extrait étant riche en composé 2,5-diformylfuran.

2. Extrait selon la revendication 1, **caractérisé en ce que** l'extrait liquide obtenu est séché de manière à obtenir un extrait sec du tourteau des graines de *Moringa peregrina* contenant plus de 50% en poids de 2,5-diformylfuran par rapport au poids total de la matière sèche.

3. Procédé d'obtention d'un extrait de graines de *Moringa peregrina* selon l'une des revendications 1 à 2, **caractérisé en ce qu'**il comprend les étapes suivantes selon lesquelles :

   a) on recueille les graines non décortiquées de *Moringa peregrina* que l'on sèche pour obtenir un taux d'humidité interne inférieur à 8% ;
   b) on presse les graines séchées de sorte à séparer l'huile du reste de la graine pour obtenir le tourteau,
   c) on broie le tourteau obtenu à l'étape b),

d) on disperse le broyat obtenu à l'étape c) dans une proportion d'environ 25% en poids de matière solide par rapport au poids total mis en oeuvre dans un solvant majoritairement alcoolique, l'alcool étant choisi parmi l'éthanol ou le méthanol avec éventuellement un co-solvant de type polyol ou eau subcritique, dans une proportion de 70 à 100% en poids d'alcool par rapport au poids total du solvant ;

e) on réalise une extraction solide-liquide, sous agitation, à une température comprise entre 16 et 30°C pendant une durée d'environ 2 heures,

f) on sépare les phases liquide et solide de manière à éliminer la phase solide et récupérer un extrait liquide de tourteau de *Moringa peregrina,* et

g) éventuellement, lorsque l'alcool est l'éthanol, on sèche l'extrait de *Moringa peregrina* liquide obtenu de manière à obtenir un extrait de *Moringa peregrina* solide.

4. Procédé selon la revendication 3, **caractérisé en ce que** le solvant majoritairement alcoolique est le solvant pur éthanol 96°.

5. Procédé selon la revendication 3, **caractérisé en ce que** l'extrait de *Moringa peregrina* liquide obtenu est purifié par distillation, microfiltration, ultrafiltration et/ou nanofiltration pour concentrer l'extrait en 2,5-diformylfuran par rapport aux matières organiques également extraites.

6. Composition cosmétique ou nutricosmétique, **caractérisée en ce qu'**elle comprend, à titre d'agent actif, une quantité efficace d'un extrait de graines de *Moringa peregrina* selon l'une des revendications 1 à 2, et un excipient physiologiquement acceptable.

7. Composition selon la revendication 6, **caractérisée en ce qu'**elle est une composition cosmétique formulée pour être appliquée topiquement sur la peau et **en ce que** l'extrait de graines de *Moringa peregrina* est présent dans la composition à une concentration de 0.002 à 20% en poids par rapport au poids total de la composition, préférentiellement de 0.01 à 10%.

8. Composition selon la revendication 6, **caractérisée en ce qu'**elle est une composition nutricosmétique formulée pour être ingérée et **en ce que** l'extrait de graines de *Moringa peregrina* est présent dans la composition à une concentration de 0.01 à 100% en poids par rapport au poids total de la composition.

9. Utilisation cosmétique ou nutricosmétique d'une composition selon l'une des revendications 6 à 8, pour améliorer l'aspect de la peau, des muqueuses ou des phanères, pour relaxer, apaiser et déstresser la peau et prévenir et/ou à lutter contre les signes cutanés du vieillissement et/ou du photo-vieillissement et prévenir les tâches de vieillesse.

**Patentansprüche**

1. Extrakt aus den Samen von *Moringa peregrina,* **dadurch gekennzeichnet, dass** er durch Fest-Flüssig-Extraktion des Presskuchens aus den nicht geschälten Samen, unter Rühren, in einem Anteil von etwa 25 Gew.-%. des Feststoffs, bezogen auf das Gesamtgewicht, durchgeführt in einem überwiegend alkoholischen Lösemittel, wobei der Alkohol ausgewählt ist aus Ethanol oder Methanol mit gegebenenfalls einem Polyol oder subkritischem Wasser als Co-Lösemittel, wobei der Alkoholanteil zwischen 70 und 100 Gew.-%, bezogen auf das Gesamtgewicht des Lösemittels, liegt, bei einer Temperatur zwischen 16 und 30°C für eine Dauer von etwa 2 Stunden, und durch Trennung der flüssigen und festen Phasen, um die feste Phase zu entfernen, erhalten ist, um einen flüssigen Extrakt aus Samen von *Moringa peregrina* zu gewinnen, wobei der Extrakt reich an der Verbindung 2,5-Diformylfuran ist.

2. Extrakt gemäß Anspruch 1, **dadurch gekennzeichnet, dass** der erhaltene flüssige Extrakt getrocknet ist, um einen trocknen Extrakt aus dem Presskuchen der Samen von *Moringa peregrina* zu erhalten, der mehr als 50 Gew.-%, bezogen auf das Gesamtgewicht des Trockenmaterials, 2,5-Diformylfuran umfasst.

3. Verfahren zur Gewinnung eines Extrakts aus den Samen von *Moringa peregrina* gemäß einem der Ansprüche 1 bis 2, **dadurch gekennzeichnet, dass** es die folgenden Schritte umfasst:

a) Sammeln und Trocknen von ungeschälten Samen von *Moringa peregrina,* um einen inneren Feuchtigkeitsgehalt von weniger als 8 % zu erhalten;

b) Pressung der getrockneten Samen, sodass sich das Öl vom Rest der Samen trennt, um den Presskuchen zu erhalten,

c) Mahlen des in Schritt b) erhaltenen Presskuchens,

d) Dispersion des in Schritt c) erhaltenen Mahlguts in einem Anteil von etwa 25 Gew.-% des Feststoffs, bezogen auf das Gesamtgewicht, durchgeführt in einem überwiegend alkoholischen Lösemittel, wobei der Alkohol ausgewählt ist aus Ethanol oder Methanol mit gegebenenfalls einem Polyol oder subkritischen Wasser als Co-Lösemittel, wobei der Alkoholanteil zwischen 70 und 100 Gew.-%, bezogen auf das Gesamtgewicht des Lösemittels, liegt,

e) Durchführung einer Fest-Flüssig-Extraktion unter Rühren bei einer Temperatur zwischen 16 und 30°C während einer Dauer von etwa 2 Stunden,

f) Trennung der flüssigen und festen Phasen, um die feste Phase zu entfernen und um einen flüssigen Extrakt aus Presskuchen von *Moringa peregrina* zu gewinnen, und

g) gegebenenfalls, wenn der Alkohol Ethanol ist, Trocknung des erhaltenen flüssigen Extrakts von *Moringa peregrina,* um einen festen Extrakt von *Moringa peregrina* zu erhalten.

4. Verfahren gemäß Anspruch 3, **dadurch gekennzeichnet, dass** das überwiegend alkoholische Lösemittel das reine Lösemittel Ethanol 96° ist.

5. Verfahren gemäß Anspruch 3, **dadurch gekennzeichnet, dass** der erhaltene flüssige Extrakt von *Moringa peregrina* durch Destillation, Mikrofiltration, Ultrafiltration und/oder Nanofiltration gereinigt wird, um den Extrakt an 2,5-Diformylfuran im Vergleich zu den ebenfalls extrahierten organischen Stoffen zu konzentrieren.

6. Kosmetische oder nutrikosmetische Zusammensetzung, **dadurch gekennzeichnet, dass** sie als Wirkstoff eine wirksame Menge eines Extrakts aus Samen von *Moringa peregrina* gemäß einem der Ansprüche 1 bis 2 und einen physiologisch annehmbaren Hilfsstoff umfasst.

7. Zusammensetzung gemäß Anspruch 6, **dadurch gekennzeichnet, dass** sie eine kosmetische Zusammensetzung ist, die formuliert ist, um topisch auf die Haut aufgetragen zu werden, und dass der Extrakt aus Samen von *Moringa peregrina* in der Zusammensetzung in einer Konzentration von 0,002 bis 20 Gew.-%, bezogen auf das Gesamtgewicht der Zusammensetzung, vorzugsweise von 0,01 bis 10 Gew.-%, vorliegt.

8. Zusammensetzung gemäß Anspruch 6, **dadurch gekennzeichnet, dass** sie eine nutrikosmetische Zusammensetzung ist, die formuliert ist, um eingenommen zu werden, und dass der Extrakt aus Samen von *Moringa peregrina* in der Zusammensetzung in einer Konzentration von 0,01 bis 100 Gew.-%, bezogen auf das Gesamtgewicht der Zusammensetzung, vorliegt.

9. Kosmetische oder nutrikosmetische Verwendung einer Zusammensetzung gemäß einem der Ansprüche 6 bis 8, um das Aussehen der Haut, der Schleimhäute oder der Anhangsgebilde zu verbessern, die Haut zu entspannen, zu beruhigen und zu entstressen und den Hautalterungserscheinungen und/oder der Fotoalterung vorzubeugen und/oder zu bekämpfen und zur Vorbeugung von Altersflecken.

## Claims

1. A *Moringa peregrina* seed extract, **characterized in that** it is obtained by solid-liquid extraction of the unshelled seed cake, with stirring, in a proportion of about 25% by weight of solid matter relative to the total weight used in a predominantly alcoholic solvent, the alcohol being chosen from ethanol or methanol optionally with a cosolvent such as a polyol or subcritical water, in a proportion of from 70% to 100% by weight of alcohol relative to the total weight of the solvent, at a temperature of between 16 and 30°C for a period of about 2 hours, and by separation of the liquid and solid phases so as to remove the solid phase and to recover a liquid extract of *Moringa peregrina* seed, said extract being rich in the compound 2,5-diformylfuran.

2. The extract as claimed in claim 1, **characterized in that** the liquid extract obtained is dried so as to obtain a dry extract of the *Moringa peregrina* seed cake containing more than 50% by weight of 2,5-diformylfuran relative to the total weight of the dry matter.

3. A process for obtaining an extract of *Moringa peregrina* seeds as claimed in either of claims 1 and 2, **characterized in that** it comprises the following steps in which:

a) the unshelled seeds of *Moringa peregrina* are collected and dried to obtain an internal moisture content of less than 8%,

b) the dried seeds are pressed so as to separate the oil from the rest of the seed, to obtain the cake,

c) the cake obtained in step b) is milled,

d) the milled material obtained in step c) is dispersed, in a proportion of about 25% by weight of solid material relative to the total weight used, in a predominantly alcoholic solvent, the alcohol being chosen from ethanol or methanol optionally with a cosolvent such as a polyol or subcritical water, in a proportion of from 70% to 100% by weight of alcohol relative to the total weight of the solvent;

e) a solid-liquid extraction is performed, with stirring, at a temperature of between 16 and 30°C over a period of about 2 hours,

f) the liquid and solid phases are separated so as to remove the solid phase and to recover a liquid Moringa peregrina cake extract, and

g) optionally, when the alcohol is ethanol, the liquid *Moringa peregrina* extract obtained is dried so as to obtain a solid *Moringa peregrina* extract.

**4.** The process as claimed in claim 3, **characterized in that** the predominantly alcoholic solvent is the solvent 96° pure ethanol.

**5.** The process as claimed in claim 3, **characterized in that** the liquid *Moringa peregrina* extract is purified by distillation, microfiltration, ultrafiltration and/or nanofiltration to concentrate the 2,5-diformylfuran of the extract relative to the organic materials also extracted.

**6.** A cosmetic or nutricosmetic composition **characterized in that** it comprises, as active agent, an effective amount of an extract of *Moringa peregrina* seeds as claimed in either of claims 1 and 2, and a physiologically acceptable excipient.

**7.** The composition as claimed in claim 6, **characterized in that** it is a cosmetic composition formulated for topical application to the skin and **in that** the extract of *Moringa peregrina* seeds is present in the composition in a concentration of from 0.002% to 20% by weight, preferentially from 0.01% to 10%, relative to the total weight of the composition.

**8.** The composition as claimed in claim 6, **characterized in that** it is a nutricosmetic composition formulated for ingestion and **in that** the extract of *Moringa peregrina* seeds is present in the composition in a concentration of from 0.01% to 100% by weight relative to the total weight of the composition.

**9.** The cosmetic or nutricosmetic use of a composition as claimed in one of claims 6 to 8, for improving the appearance of the skin, mucous membranes or the integuments, for relaxing, soothing and destressing the skin and for preventing and/or combating the signs of aging and/or photoaging of the skin, and for preventing age spots.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 296879
- FR 2776519
- FR 3076460

- KR 20130088224
- FR 2946879, de Pierre Fabre
- FR 2825267, de Chuun & Thurot

### Littérature non-brevet citée dans la description

- **OLSON M. E.** Combining Data from DNA Sequences and Morphology for a Phylogeny of Moringaceae (Brassicales). *Systematic Botany,* 2002, vol. 27 (1), 55-73
- **HASSANEIN A. M. A. ; SOQEE, A. A. et al.** Morphological and genetic diversity of Moringa oleifera and Moringa peregrina génotypes. *Horticulture, Environment and Biotechnology,* 2018, vol. 59 (2), 251-261
- **ALAKLABI A.** Genetic diversity of Moringa peregrina species in Saudi Arabia with ITS sequences. *Saudi Journal of Biological Sciences,* 2015, vol. 22, 186-190
- **MUNYANZIZA E. ; YONGAB1 K. A.** Vegetable oils/Oléagineux. *Moringa peregrina (Forssk.) Fiori,* 23 Octobre 2019, http://database.prota.org/protahtml/moringa peregrina _fr.htm
- **GOMAA N. H ; PICÓ F. X.** Seed germination, seedling traits, and seed bank of the tree Moringa peregrina (Moringaceae) in a hyper-arid environment. *American Journal of Botany,* 2011, vol. 98 (6), 1024-1030
- **NASEEF, A.A.S.** *Al- 'Ula, A study of Cultural and Social Heritage,* 1995
- **ABDEL-KADER M. S. ; HAZAZI A. M. A. ; ELMAKKI O. A. ; ALQASOUMI S. I.** survey on the traditional plants used in Al Kobah village. *Saudi Pharmaceutical Journal,* 2018, vol. 26 (6), 817-821
- **AQEEL A. A. M. ; TARIQ M. ; M OSSA J. S. ; AL-YAHYA M. A. ; SALO M. S. et al.** Plants used in Arabia Folk medicine. *Report submitted to Saudi Arabian National Cenre for Science and Technology, Riyadh, Saudi Arabia,* 1984

- **GHAZANFAR S. A.** Handbook of Arabian Médicinal Plants. CRC Press, 1994
- Végétation of the Arabian Peninsula. **GHAZANFAR S.A.** Plants of Economic Importance. Kluwer Academic Publishers, 1998, vol. 25, 241-264
- **GHAZANFAR S.A. ; RECHINGER B.** Two multi-purpose seed oils form Oman. Plants for Food and Medicin. *Paper presented at the joint meeting of the Society for Economic Botany and International Society for Ethnopharmacology,* 1996
- **AL-DABBAS M.** Antioxidant activity of different extracts from the aerial part of Moringa peregrina (Forssk.) Fiori, from Jordan. *Pakistan Journal of Pharmaceutical Sciences,* 2017, vol. 30 (6), 2151-2157
- International Nomenclature of Cosmetic Ingrédients. L'INCI Dictionary & Handbook. 2010
- **HIRATA Y. et al.** Cellular mechanism of action by a novel vasoconstrictor endothelin in cultured rat vascular smooth muscle cells. *Biochemical and Biophysical Research Commununication,* 1988, vol. 154 (3), 868-875
- **SHALINKUMAR P. et al.** H2S Médiates the Vasodilator Effect of Endothelin-1 in the Cérébral Circulation. *American Journal of Physiology. Heart and Circulatory Physioliogy,* 2018, vol. 315, 1759-1764
- **ORNISH D.** Increased Telomerase Activity and Comprehensive Lifestyle Changes: a Pilot Study. *Lancet Oncology,* 2008, vol. 9, 1048-1057
- *CHEMICAL ABSTRACTS,* 69-09-0